# EUROPEAN PATENT APPLICATION

(11) **EP 4 817 570 A2**
(43) Date of publication of application: **07.10.2026**
(21) Application number: 26200124.1
(22) Date of filing: 18.08.2015
(51) Int. Cl.: A61F 7/02

(54) **TREATMENT SYSTEMS, SMALL VOLUME APPLICATORS, AND METHODS FOR TREATING SUBMENTAL TISSUE**

(30) Priority: 19.08.2014 US 201462039213 P; 06.05.2015 US 201514705868
(62) Document number(s) of the earlier application(s) in accordance with Art. 76 EPC: 23199229.8 / 4 324 441; 15754406.5 / 3 182 918
(71) Applicant: Zeltiq Aesthetics, Inc., Pleasanton, CA 94582 (US)
(72) Inventor: YEE, Peter, San Ramon (US); COAKLEY, Joseph, Dublin (US); FRANGINEAS, George Jr., Fremont (US); HILTON, Tamara Lynn, Alameda (US)
(74) Representative: Hoffmann Eitle

(57) **Abstract**

Systems for treating a subject's tissue can include a thermally conductive cup, a tissue-receiving cavity, and a vacuum port. The vacuum port is in fluid communication with the tissue-receiving cavity to provide a vacuum for drawing the submental tissue, or other targeted tissue, into the tissue-receiving cavity. A thermal device can cool and/or heat the conductive cup such that the conductive cup non-invasively controls the temperature of subcutaneous lipid-rich cells in the tissue. A restraint apparatus can hold a the conductive cup in thermal contact with the target region.

## Description

### CROSS-REFERENCE TO RELATED APPLICATIONS

This application claims priority to U.S. Patent Application No. 14/705,868, filed May 6, 2015, and claims the benefit under 35 U.S.C. § 119(e) of U.S. Provisional Patent Application No. 62/039,213 filed August 19, 2014, which are incorporated by reference in their entireties.

### INCORPORATION BY REFERENCE OF COMMONLY-OWNED APPLICATIONS AND PATENTS

The following commonly assigned U.S. Patent Applications and U.S. Patents are incorporated herein by reference in their entireties:
U.S. Patent Publication No. 2008/0287839 entitled "METHOD OF ENHANCED REMOVAL OF HEAT FROM SUBCUTANEOUS LIPID-RICH CELLS AND TREATMENT APPARATUS HAVING AN ACTUATOR";
U.S. Patent No. 6,032,675 entitled "FREEZING METHOD FOR CONTROLLED REMOVAL OF FATTY TISSUE BY LIPOSUCTION";
U.S. Patent Publication No. 2007/0255362 entitled "CRYOPROTECTANT FOR USE WITH A TREATMENT DEVICE FOR IMPROVED COOLING OF SUBCUTANEOUS LIPID-RICH CELLS";
U.S. Patent No. 7,854,754 entitled "COOLING DEVICE FOR REMOVING HEAT FROM SUBCUTANEOUS LIPID-RICH CELLS";
U.S. Patent Publication No. 2011/0066216 entitled "COOLING DEVICE FOR REMOVING HEAT FROM SUBCUTANEOUS LIPID-RICH CELLS";
U.S. Patent Publication No. 2008/0077201 entitled "COOLING DEVICES WITH FLEXIBLE SENSORS";
U.S. Patent Publication No. 2008/0077211 entitled "COOLING DEVICE HAVING A PLURALITY OF CONTROLLABLE COOLING ELEMENTS TO PROVIDE A PREDETERMINED COOLING PROFILE";
U.S. Patent Publication No. 2009/0118722, filed October 31, 2007, entitled "METHOD AND APPARATUS FOR COOLING SUBCUTANEOUS LIPID-RICH CELLS OR TISSUE";
U.S. Patent Publication No. 2009/0018624 entitled "LIMITING USE OF DISPOSABLE SYSTEM PATIENT PROTECTION DEVICES";
U.S. Patent Publication No. 2009/0018623 entitled "SYSTEM FOR TREATING LIPID-RICH REGIONS";
U.S. Patent Publication No. 2009/0018625 entitled "MANAGING SYSTEM TEMPERATURE TO REMOVE HEAT FROM LIPID-RICH REGIONS";
U.S. Patent Publication No. 2009/0018627 entitled "SECURE SYSTEM FOR REMOVING HEAT FROM LIPID-RICH REGIONS";
U.S. Patent Publication No. 2009/0018626 entitled "USER INTERFACES FOR A SYSTEM THAT REMOVES HEAT FROM LIPID-RICH REGIONS";
U.S. Patent No. 6,041,787 entitled "USE OF CRYOPROTECTIVE AGENT COMPOUNDS DURING CRYOSURGERY";
U.S. Patent No. 8,285,390 entitled "MONITORING THE COOLING OF SUBCUTANEOUS LIPID-RICH CELLS, SUCH AS THE COOLING OF ADIPOSE TISSUE";
U.S. Patent No. 8,275,442 entitled "TREATMENT PLANNING SYSTEMS AND METHODS FOR BODY CONTOURING APPLICATIONS";
U.S. Patent Application Serial No. 12/275,002 entitled "APPARATUS WITH HYDROPHILIC RESERVOIRS FOR COOLING SUBCUTANEOUS LIPID-RICH CELLS";
U.S. Patent Application Serial No. 12/275,014 entitled "APPARATUS WITH HYDROPHOBIC FILTERS FOR REMOVING HEAT FROM SUBCUTANEOUS LIPID-RICH CELLS";
U.S. Patent Publication No. 2010/0152824 entitled "SYSTEMS AND METHODS WITH INTERRUPT/RESUME CAPABILITIES FOR COOLING SUBCUTANEOUS LIPID-RICH CELLS";
U.S. Patent No. 8,192,474 entitled "TISSUE TREATMENT METHODS";
U.S. Patent Publication No. 2010/0280582 entitled "DEVICE, SYSTEM AND METHOD FOR REMOVING HEAT FROM SUBCUTANEOUS LIPID-RICH CELLS";
U.S. Patent Publication No. 2012/0022518 entitled "COMBINED MODALITY TREATMENT SYSTEMS, METHODS AND APPARATUS FOR BODY CONTOURING APPLICATIONS";
U.S. Publication No. 2011/0238050 entitled "HOME-USE APPLICATORS FOR NON-INVASIVELY REMOVING HEAT FROM SUBCUTANEOUS LIPID-RICH CELLS VIA PHASE CHANGE COOLANTS, AND ASSOCIATED DEVICES, SYSTEMS AND METHODS";
U.S. Publication No. 2011/0238051 entitled "HOME-USE APPLICATORS FOR NON-INVASIVELY REMOVING HEAT FROM SUBCUTANEOUS LIPID-RICH CELLS VIA PHASE CHANGE COOLANTS, AND ASSOCIATED DEVICES, SYSTEMS AND METHODS";
U.S. Publication No. 2012/0239123 entitled "DEVICES, APPLICATION SYSTEMS AND METHODS WITH LOCALIZED HEAT FLUX ZONES FOR REMOVING HEAT FROM SUBCUTANEOUS LIPID-RICH CELLS ";
U.S. Patent Application Serial No. 13/830,413 entitled "MULTI-MODALITY TREATMENT SYSTEMS, METHODS AND APPARATUS FOR ALTERING SUBCUTANEOUS LIPID-RICH TISSUE";
U.S. Patent Application Serial No. 13/830,027 entitled "TREATMENT SYSTEMS WITH FLUID MIXING SYSTEMS AND FLUID-COOLED APPLICATORS AND METHODS OF USING THE SAME";
U.S. Patent Application Serial No. 11/528,225 entitled "COOLING DEVICE HAVING A PLURALITY OF CONTROLLABLE COOLING ELEMENTS TO PROVIDE A PREDETERMINED COOLING PROFILE;" and
U.S. Patent No. 8,285,390 entitled "MONITORING THE COOLING OF SUBCUTANEOUS LIPID-RICH CELLS, SUCH AS THE COOLING OF ADIPOSE TISSUE."

### TECHNICAL FIELD

The present disclosure relates generally to treatment systems for cooling and/or cooling targeted regions. Several embodiments are directed to treatment systems with non-invasive applicators that hold and cool/heat relatively small volumes of tissue. Several embodiments can also include restraint apparatuses for holding non-invasive applicators in thermal contact with patients.

### BACKGROUND

Excess body fat, or adipose tissue, may be present at various locations of a subject's body and may detract from personal appearance. Excess subcutaneous fat under the chin and/or around the neck can be cosmetically unappealing and, in some instances, can produce a "double chin." A double chin can cause stretching and/or sagging of skin and may also result in discomfort. Excess adipose tissue in superficial fat compartments can produce loose facial structures, such as loose jowls, that also cause an undesirable appearance. Excess body fat can also be located at the abdomen, thighs, buttocks, knees, and arms, as well as other locations.

Aesthetic improvement of the human body often involves the selective removal of adipose tissue. Invasive procedures (e.g., liposuction), however, tend to be associated with relative high costs, long recovery times, and increased risk of complications. Injection of drugs for reducing adipose tissue, such as submental or facial adipose tissue, can cause significant swelling, bruising, pain, numbness, and/or induration. Conventional non-invasive treatments for reducing adipose tissue often include regular exercise, application of topical agents, use of weight-loss drugs, dieting, or a combination of these treatments. One drawback of these non-invasive treatments is that they may not be effective or even possible under certain circumstances. For example, when a person is physically injured or ill, regular exercise may not be an option. Topical agents and orally administered weight-loss drugs are not an option if, as another example, they cause an undesirable reaction (e.g., an allergic or negative reaction). Additionally, non-invasive treatments may be ineffective for selectively reducing specific regions of adiposity. For example, localized fat loss around the neck, jaw, cheeks, etc. often cannot be achieved using general or systemic weight-loss methods. Accordingly, conventional invasive and non-invasive treatments are not suitable for many subjects and cannot effectively target certain regions of adipose tissue.

### SUMMARY OF TECHNOLOGY

Systems for treating a subject's tissue can include a thermally conductive cup, a tissue-receiving cavity, and a vacuum port. The vacuum port can be in fluid communication with the tissue-receiving cavity to provide a vacuum for drawing the submental tissue, or other targeted tissue, into the tissue-receiving cavity. The system can cool and/or heat the conductive cup such that the conductive cup non-invasively controls the temperature of subcutaneous lipid-rich cells in the tissue. A restraint apparatus can hold the thermally conductive cup in thermal contact with a patient's tissue.

At least some embodiments are apparatuses for treating a subject's submental tissue and can include a thermally conductive cup, at least one vacuum port, and a thermal device. The thermally conductive cup can include a first sidewall, a second sidewall, and a bottom. The vacuum port can be in fluid communication with a tissue-receiving cavity of the cup to provide a vacuum for drawing the submental tissue into the tissue-receiving cavity. The tissue-receiving cavity can be sufficiently shallow to allow the subject's submental tissue to occupy substantially the entire tissue-receiving cavity when the vacuum is drawn via the vacuum port. The thermal device can be in thermal communication with the conductive cup. The thermal device can be configured to cool the conductive cup such that the first sidewall, second sidewall, and bottom together non-invasively cool subcutaneous lipid-rich cells in the submental tissue. For example, the subcutaneous lipid-rich cells can be cooled an amount sufficient to be biologically effective in damaging and/or reducing the subcutaneous lipid-rich cells or other targeted cells.

The first sidewall, second sidewall, and bottom can be positioned to absorb heat from the submental tissue to damage and/or reduce the lipid-rich cells, which are in a subcutaneous layer of adipose tissue, in number and/or size to an extent while non-lipid-rich cells deeper than the subcutaneous layer of adipose tissue are not reduced in number and/or size to the extent. In some embodiments, the apparatus can include a pressurization device in fluid communication with the tissue-receiving cavity via the vacuum port. A controller can include instructions for causing the apparatus to hold the submental tissue in the tissue-receiving cavity using suction provided by the pressurization device.

The conductive cup can be in thermal contact with most of the subject's skin at the subject's submental region when the tissue-receiving cavity is partially or completely filled with the subject's tissue. The conductive cup can include a conductive surface (e.g., metal surface) that faces the tissue-receiving cavity and has an area equal to or less than about, for example, 40 cm². In some embodiments, the conductive cup can include a smooth thermally conductive surface that extends continuously along the first sidewall, second sidewall, and bottom.

The tissue-receiving cavity can be dimensioned to receive most of the subject's skin located at the submental region of the subject. In some embodiments, the tissue-receiving cavity has a length between opposing end walls of the conductive cup, a width between the first and second sidewalls, and a depth between an opening of the tissue-receiving cavity and the bottom of the conductive cup. The depth is substantially uniform along most of the length of the tissue-receiving cavity.

A liner assembly can line the conductive cup such that the liner assembly is positioned between the subject's tissue in the tissue-receiving cavity and the conductive cup. The linear assembly can be made of plastic, rubber, or other suitable material and can carry and/or include one or more sensors.

In some embodiments, an apparatus for treating a subject's tissue includes a submental vacuum applicator. The submental vacuum applicator can include a tissue-receiving cavity, a contoured lip, and a thermal device. The contoured lip can define a mouth of the tissue-receiving cavity and can include first and second arcuate lip portions. The contoured lip can be configured to engage a submental area of the subject such that mostly submental tissue extends through the mouth and fills substantially all of the tissue-receiving cavity while the submental vacuum applicator draws a vacuum and the first and second arcuate lip portions surround at least a portion of the subject's body. The thermal device can be positioned to be in thermal contact with the submental tissue in the tissue-receiving cavity. The thermal device is operable to non-invasively cool subcutaneous lipid-rich cells in the submental tissue an amount sufficient to be biologically effective in damaging, reducing, and/or otherwise affecting the subcutaneous lipid-rich cells.

The apparatus can include a controller with instructions for causing the submental vacuum applicator to cool a conductive cup such that the submental vacuum applicator non-invasively cools the subcutaneous lipid-rich cells to a temperature less than about predetermined temperature (e.g., about 0°C, about - 1.8°C, etc.). The controller can include one or more processors, memory, power supplies, or other electrical components.

The tissue-receiving cavity can include a first end, a second end, and a central section extending between the first and second ends. The central section has a curved longitudinal axis and a substantially uniform maximum depth along most of the curved longitudinal axis. In one embodiment, the curved longitudinal axis has the same curvature as a curvature of at least one of the first and second arcuate lip portions. In one embodiment, the tissue-receiving cavity has a substantially uniform maximum depth along most of a longitudinal length of the tissue-receiving cavity.

The apparatus can further include a vacuum source fluidically coupled to the tissue-receiving cavity. The vacuum source can be configured to provide sufficient vacuum to draw the submental tissue toward a bottom of the tissue-receiving cavity to bring the submental tissue into thermal contact with a concave metal heat-exchanging surface of the submental vacuum applicator.

The submental vacuum applicator, in some embodiments, can include an applicator unit and a liner assembly removably attached to the applicator unit. In other embodiments, the applicator unit can be used without any liner assembly.

In further embodiments, a method of non-invasively cooling a submental region of a subject includes placing a submentum applicator on the subject. The submentum applicator includes a vacuum cup and a tissue-receiving cavity. Submental tissue can be drawn through the tissue-receiving cavity and into thermal contact with a section of the vacuum cup located at a bottom of the tissue-receiving cavity. Heat can be conductively extracted from the submental tissue by the submentum applicator so as to cool the submental tissue an amount sufficient to be biologically effective in selectively damaging and/or reducing subcutaneous submental lipid-rich cells. Heat can be repeatedly extracted from the subcutaneous submental tissue until desired tissue reduction is achieved. In some embodiments, a sufficient amount of heat can be conductively extracted from the submental tissue to visibly reduce a double chin of the subject.

The conductive extraction of heat can include conductively cooling an area of the subject's submental skin that is equal to or less than about 40 cm². In some embodiments, a concave heat-exchanging surface of the applicator can be cooled to a temperature equal to or less than a selected temperature (e.g., 0°C). In some embodiments, most of a heat-exchanging surface of a conductive cup of the vacuum applicator can be cooled to a temperature equal to or less than about -5°C. The submental tissue can be pulled into the tissue-receiving cavity such that the tissue-receiving cavity is filled mostly with submental tissue. In some embodiments, a vacuum can be drawn to pull the submental tissue into the tissue-receiving cavity and can result in a relatively large contact area for heat transfer with the target tissue.

In some embodiments, a system includes a restraint apparatus configured to hold a subject's head. The restraint apparatus can include an adjustable pillow and restraints. The pillow can include a head cradle portion operable to controllably adjust tilt of a subject's head. The restraints are coupleable to the pillow such that the restraints hold a tissue-cooling apparatus in thermal contact with the subject's submental region while the subject's head is supported at a desired tilt by the head cradle portion.

The system can further include a tissue-cooling apparatus configured to be connected to the pillow by the restraints. The restraints and pillow cooperate to inhibit movement of the tissue-cooling apparatus relative to the subject's submental region while the tissue-cooling apparatus non-invasively cools subcutaneous lipid-rich cells at the subject's submental region. For example, the subcutaneous lipid-rich cells can be cooled an amount sufficient to be biologically effective in damaging and/or reducing the subcutaneous lipid-rich cells. In some embodiments, the pillow and restraints can be configured to cooperate to inhibit movement of the subject's head while the tissue-cooling apparatus transcutaneously cools the subject's submental region.

The restraint apparatus, in some embodiments, further includes a head adjuster device and a neck adjuster device for reconfiguring the pillow. The head adjuster device is operable to reconfigure the head cradle portion of the pillow to achieve the desired tilt of the subject's head. The neck adjuster device is operable to reconfigure a neck support portion of the pillow to achieve desired neck tilt. In one embodiment, the head adjuster device has a bladder that expands to increase a slope of a tilted support surface of the head cradle portion so as to tilt the subject's head forward. In another embodiment, the head adjuster device includes a bladder located within an expandable opening of the pillow. The bladder can be inflated to expand at least a portion of the pillow to adjust head tilt of the subject.

The pillow, in some embodiments, can include a neck support portion which is positioned to be located under the subject's neck when the subject's head is supported by the head cradle portion. A neck adjuster device is operable to move the neck support portion against the subject's neck to adjust neck tilt of the subject. In some embodiments, the pillow includes side portions positionable on opposite sides of the subject's head. The restraint apparatus can be configured to extend across at least a portion of the subject's body to hold the tissue-cooling apparatus in thermal contact with the subject's submental region. The pillow can include a shoulder support portion and a neck support portion positioned between the shoulder support portion and the head cradle portion. The neck support portion can be moved relative to the shoulder support portion and/or head cradle portion to push against the posterior region of the subject's neck.

The restraint apparatus, in some embodiments, can include a cradle adjuster device and a neck adjuster device. The cradle adjuster device can have a first expandable element that can expand a sufficient amount to increase forward tilt of the subject's head. The neck adjuster device can have a second expandable element that can expand so as to cause the neck support portion to push against the subject's neck (e.g., a posterior region of the subject's neck) when the posterior region of the subject's head rests on the head cradle portion. The first and second expandable elements can be independently expanded to independently move different regions of the pillow.

In some embodiments, a system configured to position a subject's body includes an adjustable pillow configured to support the subject's head. The pillow can include a head cradle, a head adjuster device, and a neck adjuster device. The head cradle has side portions positioned to contact opposite sides of a subject's head received by the head cradle to inhibit movement of the subject's head. The head adjuster device is operable to tilt the head cradle portion to achieve desired tilt of the subject's head. The neck adjuster device is operable to reconfigure a neck support portion of the pillow such that the neck support portion pushes against the subject's neck to achieve desired neck tilt.

The system can further include one or more restraints configured to hold a tissue-cooling apparatus in thermal contact with the subject's submental region while the head cradle inhibits movement of the subject's head relative to the tissue-cooling apparatus. In one embodiment, the restraints have an open configuration for allowing the subject's head to be moved into or out of the head cradle and a closed configuration for keeping the subject's head in the head cradle. In some embodiments, the restraints can be tensioned to pull the tissue-cooling apparatus toward the subject's submental region. The restraint apparatus can include hook and loop fastener that detachably couples the restraints to the pillow. For example, the loop fastener can be part of or attached to the pillow. The hook fastener can be part of or attached to the restraints. In other embodiments, the system can include a harness, straps, fasteners (e.g., buckles, snaps, etc.), and/or other coupling means for holding the subject's body, tissue-cooling apparatus, or the like.

The system, in some embodiments, further includes a tissue-cooling apparatus and at least one restraint. The tissue-cooling apparatus is configured to non-invasively cool subcutaneous lipid-rich cells at the subject's submental region an amount sufficient to be biologically effective in damaging and/or reducing the subcutaneous lipid-rich cells. The restraint is detachably coupleable to the pillow and detachably coupleable to the tissue-cooling apparatus. The tissue-cooling apparatus can be a handheld device with one or more thermoelectric cooling devices (e.g., Peltier devices), cooling channels, sensors, electrical components (e.g., circuitry, controllers, etc.), and/or other components.

At least some treatment systems disclosed herein can include a restraint apparatus that includes an adjustable pillow and means for stabilizing a tissue-cooling apparatus. In some embodiments, the means for stabilizing the tissue-cooling apparatus can include one or more restraints. The pillow can include a head cradle portion and means for controllably adjusting tilt of the subject's head and/or neck supported by the adjustable pillow. In one embodiment, the means for controllably adjusting tilt of the subject's head and/or neck includes a bladder insertable into the adjustable pillow and a pump connected to the bladder. In one embodiment, the means for controllably adjusting tilt of the subject's head and/or neck includes a cradle adjuster device and a neck adjuster device. The cradle adjuster device is positionable in the head cradle portion and can be expanded to increase forward tilt of the subject's head. The neck adjuster device can be expanded to cause a neck support portion of the pillow to push against the subject's neck when the subject's head is supported by the head cradle portion.

The head cradle portion, in some embodiments, can include side portions spaced apart to be positioned on opposite sides of the subject's head. The means for stabilizing the tissue-cooling apparatus can include restraints connectable to the side portions such that the one or more of the restraints extend across the subject's body to hold the tissue-cooling apparatus in thermal contact with the subject's submental region. In some embodiments, the means for stabilizing the tissue-cooling apparatus includes a retention system with one or more restraints, straps, or other coupling features.

### BRIEF DESCRIPTION OF THE DRAWINGS

In the drawings, identical reference numbers identify similar elements or acts.
Figure 1 is a partially schematic, isometric view of a treatment system for non-invasively affecting target regions of a subject in accordance with an embodiment of the technology.
Figure 2 is a cross-sectional view of a connector taken along line 2-2 of Figure 1.
Figure 3 is a side view of an applicator applied to a subject while the subject's head is supported by an adjustable pillow in accordance with embodiments of the technology.
Figure 4 is a cross-sectional view of the applicator and the subject's tissue taken along line 4-4 of Figure 3.
Figure 5 is an isometric view of an applicator unit suitable for use with the system of Figure 1 in accordance with embodiments of the technology.
Figure 6 is an exploded isometric view of the applicator unit of Figure 5.
Figure 7 is a top view of the applicator unit of Figure 5 in accordance with embodiments of the technology.
Figure 8 is a cross-sectional view of the applicator unit taken along line 8-8 of Figure 7.
Figure 9 is a cross-sectional view of the applicator unit taken along line 9-9 of Figure 7.
Figures 9A and 9B are cross-sectional views of vacuum cups in accordance with embodiments of the technology.
Figure 10 is an isometric view of a liner assembly in accordance with embodiments of the technology.
Figure 11 is a top view of the liner assembly of Figure 10.
Figure 12 is a cross-sectional view of the liner assembly taken along line 12-12 of Figure 11.
Figure 13 shows an applicator ready to be placed against a subject's skin in accordance with embodiments of the technology.
Figure 14 shows the applicator contacting the subject's skin.
Figure 15 is a cross-sectional view of the applicator before tissue has been drawn into a tissue-receiving cavity of the applicator.
Figure 16 is a cross-sectional view of the applicator after tissue has been drawn into the tissue-receiving cavity.
Figure 17 shows the subject after cryotherapy has been performed.
Figures 18-21 are isometric views of vacuum cups in accordance with embodiments of the present technology.
Figure 22 is an isometric view of an applicator unit in accordance with embodiments of the technology.
Figure 23A is an isometric view of an applicator in accordance with embodiments of the technology.
Figure 23B is a side view of the applicator and connector of Figure 23A.
Figure 23C is an exploded isometric view of the applicator and connector of Figure 23A in accordance with embodiments of the technology.
Figure 23D is a cross-section view of the applicator of Figure 23A.
Figure 24 is an isometric view of a head support assembly in accordance with embodiments of the present technology.
Figure 25 is a cross-sectional view of the head support assembly taken along line 25-25 of Figure 27 when a pillow is in an unexpanded lowered configuration.
Figure 26 is a cross-sectional view of the head support assembly taken along line 26-26 of Figure 27 when the pillow is in an expanded raised configuration.
Figure 27 is a top view of a pillow in accordance with embodiments of the present technology.
Figure 28 is a front view of the pillow of Figure 27.
Figure 29 is a side view of the pillow of Figure 27.
Figures 30 and 31 are top views of adjuster devices in accordance with embodiments of the present technology.
Figure 32 is a top view of an adjustable pillow supporting the subject's head and restraints ready to be coupled to the pillow.
Figure 33 is a top view of the restraint apparatus holding the applicator in thermal contact with the subject.
Figure 34 is a side view of the restraint apparatus holding the applicator in thermal contact with the subject.
Figure 35 is a schematic block diagram illustrating subcomponents of a controller in accordance with embodiments of the technology.

### DETAILED DESCRIPTION

### A. Overview

The present disclosure describes treatment systems, applicators, and methods for affecting targeted sites. Several embodiments are directed to non-invasive systems that cool/heat relatively small regions or volumes of tissue, including submental tissue, neck tissue, etc. The systems can help position the patient's body to enhance treatment. Several of the details set forth below are provided to describe the following examples and methods in a manner sufficient to enable a person skilled in the relevant art to practice, make, and use them. Several of the details and advantages described below, however, may not be necessary to practice certain examples and methods of the technology. Additionally, the technology may include other examples and methods that are within the scope of the technology but are not described in detail.

At least some embodiments are systems for treating a subject's tissue and can include a thermally conductive cup, a tissue-receiving cavity, and a vacuum port. The vacuum port is in fluid communication with the tissue-receiving cavity to provide a vacuum for drawing the submental tissue into the tissue-receiving cavity. The thermal device can cool or heat the conductive cup such that the conductive cup non-invasively cools subcutaneous lipid-rich cells in the submental tissue an amount sufficient to affect targeted tissue. A restraint system can hold the conductive cup at the treatment site to enhance treatment.

In some embodiments, an apparatus for treating a subject's tissue includes a thermally conductive element, a vacuum port, and a thermal device for heating/cooling the conductive element. The conductive element can be a metal cup with sidewalls and a bottom. The vacuum port can be in fluid communication with a tissue-receiving cavity defined by the metal cup to provide a vacuum for drawing tissue into the tissue-receiving cavity. When the thermal device heats or cools the conductive cup, the heated/cooled sidewalls and/or bottom can non-invasively heat/cool subcutaneous lipid-rich cells in the submental tissue, which is located in the tissue-receiving cavity, an amount sufficient to be biologically effective in altering the subcutaneous lipid-rich cells. The thermal device can include one or more cooling/heating elements (e.g., resistive heaters, fluid-cooled elements, Peltier devices, etc.), controllers, sensors, or combinations thereof.

The term "treatment system", as used generally herein, refers to cosmetic or medical treatment systems, as well as any treatment regimens or medical device usage. Several embodiments of treatment system disclosed herein can reduce or eliminate excess adipose tissue or other undesirable tissue treatable using cryotherapy. The treatment systems can be used at various locations, including, for example, a subject's face, neck, abdomen, thighs, buttocks, knees, back, arms, ankles, and other areas. For example, a submental region can be treated to visibly reduce or eliminate a double chin or other unwanted tissue.

Some of the embodiments disclosed herein can be for cosmetically beneficial alterations of target regions. Some cosmetic procedures may be for the sole purpose of altering the target region to conform to a cosmetically desirable look, feel, size, shape and/or other desirable cosmetic characteristic or feature. Accordingly, at least some embodiments of the cosmetic procedures can be performed without providing an appreciable therapeutic effect (e.g., no therapeutic effect). For example, some cosmetic procedures may not include restoration of health, physical integrity, or the physical well-being of a subject. The cosmetic methods can target subcutaneous regions to change a subject's appearance and can include, for example, procedures performed on subject's submental region, face, neck, ankle region, or the like. In other embodiments, however, cosmetically desirable treatments may have therapeutic outcomes (whether intended or not), such as psychological benefits, alteration of body hormones levels (by the reduction of adipose tissue), etc.

Reference throughout this specification to "one example," "an example," "one embodiment," or "an embodiment" means that a particular feature, structure, or characteristic described in connection with the example is included in at least one example of the present technology. Thus, the occurrences of the phrases "in one example," "in an example," "one embodiment," or "an embodiment" in various places throughout this specification are not necessarily all referring to the same example. Furthermore, the particular features, structures, routines, stages, or characteristics may be combined in any suitable manner in one or more examples of the technology. The headings provided herein are for convenience only and are not intended to limit or interpret the scope or meaning of the technology.

### B. Cryotherapy

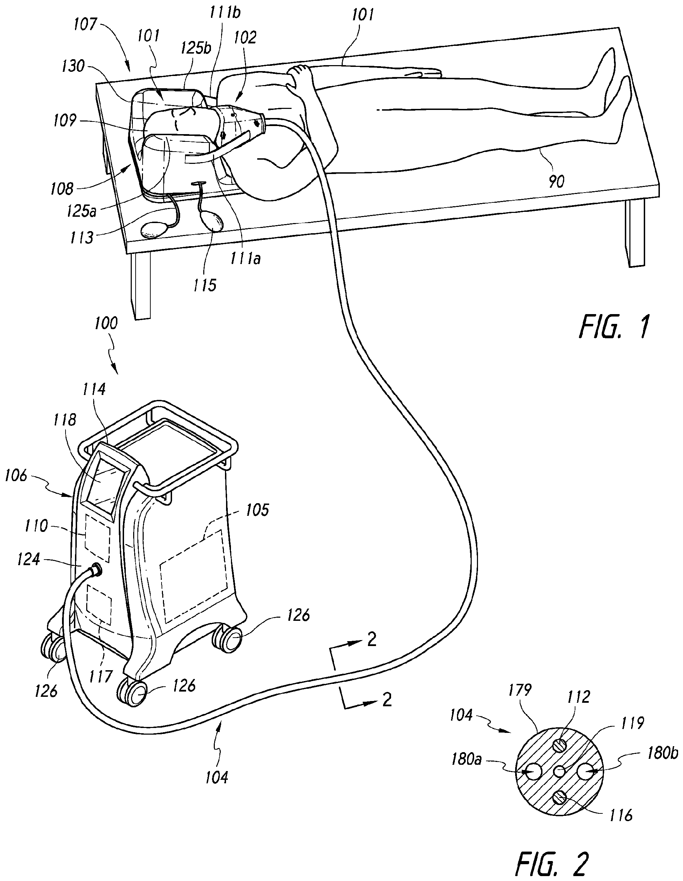

Figure 1 and the following discussion provide a brief, general description of a treatment system 100 in accordance with some embodiments of the technology. The treatment system 100 can be a temperature-controlled system for exchanging heat with a subject 101 and can include a non-invasive tissue-cooling apparatus in the form of an applicator 102 ("applicator 102") configured to selectively cool/heat tissue to reduce and/or eliminate targeted tissue to achieve a desired overall appearance. The illustrated treatment system 100 includes a restraint apparatus 107 configured to hold the applicator 102 generally under the subject's chin to reduce or eliminate submental lipid-rich fat cells so as to reduce or eliminate, for example, a double chin. Skin, muscle, connective tissue of the neck and/or face, or other non-targeted tissue can be generally unaffected. The applicator 102 can also treat relatively small volumes of tissue at other locations.

The treatment system 100 can perform medical treatments to provide therapeutic effects and/or cosmetic procedures for cosmetically beneficial effect. Without being bound by theory, the selective effect of cooling is believed to result in, for example, membrane disruption, cell shrinkage, disabling, disrupting, damaging, destroying, removing, killing and/or other methods of lipid-rich cell alteration. Such alteration is believed to stem from one or more mechanisms acting alone or in combination. It is thought that such mechanism(s) trigger an apoptotic cascade, which is believed to be the dominant form of lipid-rich cell death by non-invasive cooling. In any of these embodiments, the effect of tissue cooling can be the selectively reduction of lipid-rich cells by a desired mechanism of action, such as apoptosis, lipolysis, or the like. In some procedures, the applicator 102 can cool the tissue of the subject 101 to a temperature in a range of from about -25°C to about 20°C. In other embodiments, the cooling temperatures can be from about -20°C to about 10°C, from about -18°C to about 5°C, from about -15°C to about 5°C, or from about -15°C to about 0°C. In further embodiments, the cooling temperatures can be equal to or less than -5°C, -10°C, -15°C, or in yet another embodiment, from about - 15°C to about -25°C. Other cooling temperatures and temperature ranges can be used.

Apoptosis, also referred to as "programmed cell death", is a genetically-induced death mechanism by which cells self-destruct without incurring damage to surrounding tissues. An ordered series of biochemical events induce cells to morphologically change. These changes include cellular blebbing, loss of cell membrane asymmetry and attachment, cell shrinkage, chromatin condensation and chromosomal DNA fragmentation. Injury via an external stimulus, such as cold exposure, is one mechanism that can induce cellular apoptosis in cells. Nagle, W.A., Soloff, B.L., Moss, A.J. Jr., Henle, K.J. "Cultured Chinese Hamster Cells Undergo Apoptosis After Exposure to Cold but Nonfreezing Temperatures" Cryobiology 27, 439-451 (1990).

One aspect of apoptosis, in contrast to cellular necrosis (a traumatic form of cell death causing local inflammation), is that apoptotic cells express and display phagocytic markers on the surface of the cell membrane, thus marking the cells for phagocytosis by macrophages. As a result, phagocytes can engulf and remove the dying cells (e.g., the lipid-rich cells) without eliciting an immune response. Temperatures that elicit these apoptotic events in lipid-rich cells may contribute to long-lasting and/or permanent reduction and reshaping of subcutaneous adipose tissue.

One mechanism of apoptotic lipid-rich cell death by cooling is believed to involve localized crystallization of lipids within the adipocytes at temperatures that do not induce crystallization in non-lipid-rich cells. The crystallized lipids selectively may injure these cells, inducing apoptosis (and may also induce necrotic death if the crystallized lipids damage or rupture the bi-lipid membrane of the adipocyte). Another mechanism of injury involves the lipid phase transition of those lipids within the cell's bi-lipid membrane, which results in membrane disruption or dysfunction, thereby inducing apoptosis. This mechanism is well-documented for many cell types and may be active when adipocytes, or lipid-rich cells, are cooled. Mazur, P., "Cryobiology: the Freezing of Biological Systems" Science, 68: 939-949 (1970); Quinn, P.J., "A Lipid Phase Separation Model of Low Temperature Damage to Biological Membranes" Cryobiology, 22: 128-147 (1985); Rubinsky, B., "Principles of Low Temperature Preservation" Heart Failure Reviews, 8, 277-284 (2003). Other possible mechanisms of adipocyte damage, described in U.S. Patent No. 8,192,474, relate to ischemia/reperfusion injury that may occur under certain conditions when such cells are cooled as described herein. For instance, during treatment by cooling as described herein, the targeted adipose tissue may experience a restriction in blood supply and thus be starved of oxygen due to isolation as a result of applied pressure, cooling which may affect vasoconstriction in the cooled tissue, or the like. In addition to the ischemic damage caused by oxygen starvation and the buildup of metabolic waste products in the tissue during the period of restricted blood flow, restoration of blood flow after cooling treatment may additionally produce reperfusion injury to the adipocytes due to inflammation and oxidative damage that is known to occur when oxygenated blood is restored to tissue that has undergone a period of ischemia. This type of injury may be accelerated by exposing the adipocytes to an energy source (via, e.g., thermal, electrical, chemical, mechanical, acoustic, or other means) or otherwise increasing the blood flow rate in connection with or after cooling treatment as described herein. Increasing vasoconstriction in such adipose tissue by, e.g., various mechanical means (e.g., application of pressure or massage), chemical means or certain cooling conditions, as well as the local introduction of oxygen radical-forming compounds to stimulate inflammation and/or leukocyte activity in adipose tissue may also contribute to accelerating injury to such cells. Other yet-to-be understood mechanisms of injury may exist.

In addition to the apoptotic mechanisms involved in lipid-rich cell death, local cold exposure is also believed to induce lipolysis (i.e., fat metabolism) of lipid-rich cells and has been shown to enhance existing lipolysis which serves to further increase the reduction in subcutaneous lipid-rich cells. Vallerand, A.L., Zamecnik. J., Jones, P.J.H., Jacobs, I. "Cold Stress Increases Lipolysis, FFA Ra and TG/FFA Cycling in Humans" Aviation, Space and Environmental Medicine 70, 42-50 (1999).

One expected advantage of the foregoing techniques is that the subcutaneous lipid-rich cells in the target region can be reduced generally without collateral damage to non-lipid-rich cells in the same region. In general, lipid-rich cells can be affected at low temperatures that do not affect non-lipid-rich cells. As a result, lipid-rich cells, such as those associated with highly localized adiposity (e.g., submental adiposity, submandibular adiposity, facial adiposity, etc.), can be affected while non-lipid-rich cells (e.g., myocytes) in the same generally region are not damaged. The unaffected non-lipid-rich cells can be located underneath lipid-rich cells (e.g., cells deeper than a subcutaneous layer of fat), in the dermis, in the epidermis, and/or at other locations.

In some procedures, the treatment system 100 can remove heat from underlying tissue through the upper layers of tissue and create a thermal gradient with the coldest temperatures near the cooling surface, or surfaces, of the applicator 102 (i.e., the temperature of the upper layer(s) of the skin can be lower than that of the targeted underlying target cells). It may be challenging to reduce the temperature of the targeted cells low enough to be destructive to these target cells (e.g., induce apoptosis, cell death, etc.) while also maintaining the temperature of the upper and surface skin cells high enough so as to be protective (e.g., nondestructive). The temperature difference between these two thresholds can be small (e.g., approximately, 5°C to about 10°C, less than 10°C, less than 15°C, etc.). Protection of the overlying cells (e.g., typically water-rich dermal and epidermal skin cells) from freeze damage during dermatological and related aesthetic procedures that involve sustained exposure to cold temperatures may include improving the freeze tolerance and/or freeze avoidance of these skin cells by using, for example, cryoprotectants for inhibiting or preventing such freeze damage.

### C. Treatment Systems

Figure 1 shows the treatment system 100 that can include the applicator 102, the restraint apparatus 107, a connector 104, and a control module 106 for controlling operation of the applicator 102. The applicator 102 can conform closely to the contours of the subject's body. The restraint apparatus 107 can include a head support assembly 108 for holding the subject's head 109 and restraints 111a, 111b (collectively "restraints 111") for connecting the applicator 102 to the head support assembly 108. The head support assembly 108 can include an adjustable pillow 130 with independently movable features capable of positioning different regions of the subject's body any number of times. After completing a cryotherapy procedure, the restraints 111 can be detached from the pillow 130 to release the subject 101.

Figure 1 shows the head support assembly 108 holding the subject's head 109 at a preferred position for treating submental tissue, reducing the likelihood of unintentional movement of the applicator 102 relative to the treatment site, to enhance a treatment. In some embodiments, the head support assembly 108 can include a head adjuster device 113 and a neck adjuster device 115. The head adjuster device 113 can be operated to adjust the forward tilt of the subject's head 109, and the neck adjuster device 115 can be operated to adjust the position of the subject's neck. The restraints 111 can be tensioned to pull the applicator 102 toward the subject's submental region and hold the subject's shoulders against side portions 125a, 125b (collectively "side portions 125"). The restraints 111 can be sufficiently tensioned to inhibit movement of the applicator 102 relative to the treatment site while the applicator 102 non-invasively cools the treatment site.

The connector 104 extends from the control module 106 to the applicator 102. Figure 2 is a cross-sectional view of the connector 104 taken along line 2-2 of Figure 1. Referring to Figure 1, the connector 104 can provide suction for drawing tissue into the applicator 102 and energy (e.g., electrical energy) and fluid (e.g., coolant) from the control module 106 to the applicator 102. Referring now to Figure 2, the connector 104 can include a main body 179, a supply fluid line or lumen 180a ("supply fluid line 180a"), and a return fluid line or lumen 180b ("return fluid line 180b"). The main body 179 may be configured (via one or more adjustable joints) to "set" in place for the treatment of the subject 101. The supply and return fluid lines 180a, 180b can be conduits comprising, in whole or in part, polyethylene, polyvinyl chloride, polyurethane, and/or other materials that can accommodate circulating coolant, such as water, glycol, synthetic heat transfer fluid, oil, a refrigerant, and/or any other suitable heat conducting fluid. In one embodiment, each fluid line 180a, 180b can be a flexible hose surrounded by the main body 179. The connector 104 can also include one or more electrical lines 112 for providing power to the applicator 102 and one or more control lines 116 for providing communication between the control module 106 (Figure 1) and the applicator 102 (Figure 1). To provide suction, the connector 104 can include one or more vacuum lines 119. In various embodiments, the connector 104 can include a bundle of fluid conduits, a bundle of power lines, wired connections, vacuum lines, and other bundled and/or unbundled components selected to provide ergonomic comfort, minimize unwanted motion (and thus potential inefficient removal of heat from the subject 101), and/or to provide an aesthetic appearance to the treatment system 100.

Referring again to Figure 1, the control module 106 can include a fluid system 105 (illustrated in phantom line), a power supply 110 (illustrated in phantom line), and a controller 114 carried by a housing 124 with wheels 126. The fluid system 105 can include a fluid chamber and a refrigeration unit, a cooling tower, a thermoelectric chiller, heaters, or any other device capable of controlling the temperature of coolant in the fluid chamber. The coolant can be continuously or intermittently delivered to the applicator 102 via the supply fluid line 180a (Figure 2) and can circulate through the applicator 102 to absorb heat. The coolant, which has absorbed heat, can flow from the applicator 102 back to the control module 106 via the return fluid line 180b (Figure 2). For warming periods, the control module 106 can heat the coolant such that warm coolant is circulated through the applicator 102. Alternatively, a municipal water supply (e.g., tap water) can be used in place of or in conjunction with the control module 106.

A pressurization device 117 can provide suction to the applicator 102 via the vacuum line 119 (Figure 2) and can include one or more pumps, vacuum sources, or the like. Air pressure can be controlled by a regulator located between the pressurization device 117 and the applicator 102. If the vacuum level is too low, tissue may not be drawn adequately (or at all) into the applicator 102. If the vacuum level is too high, undesirable discomfort to the patient 101 and/or tissue damage could occur. The control module 106 can control the vacuum level to draw tissue into the applicator 102 while maintaining a desired level of comfort. According to certain embodiments, approximately 0.5 inch Hg, 1 inch Hg, 2 inches Hg, 3 inches Hg, or 5 inches Hg vacuum is applied to draw facial or neck tissue into the applicator 102. Other vacuum levels can be selected based on the characteristics of the tissue and desired level of comfort.

The power supply 110 can provide a direct current voltage for powering electrical elements (e.g., thermal devices) of the applicator 102 via the line 112 (Figure 2). An operator can use an input/output device in the form of a screen 118 ("input/output device 118") of the controller 114 to control operation of the treatment system 100, and the input/output device 118 can display the state of operation of the treatment system 100 and/or progress of a treatment protocol. In some embodiments, the controller 114 can exchange data with the applicator 102 via the line 116 (Figure 2), a wireless communication link, or an optical communication link and can monitor and adjust treatment based on, without limitation, one or more treatment profiles and/or patient-specific treatment plans, such as those described, for example, in commonly assigned U.S. Patent No. 8,275,442. Each treatment profile and treatment plan can include one or more segments, and each segment can include temperature profiles, vacuum levels, and/or specified durations (e.g., 1 minute, 5 minutes, 10 minutes, 20 minutes, 30 minutes, 1 hour, 2 hours, etc.). Additionally, if the treatment system 100 includes multiple applicators, a treatment profile can include specific profiles for each applicator to concurrently or sequentially treat multiple treatment sites, including, but not limited to, sites along the subject's face and/or neck (e.g., submental sites, submandibular sites, etc.), abdomen, thighs, buttocks, knees, back, arms, ankle region, or other treatment sites. In some embodiments, the controller 114 can be incorporated into the applicator 102 or another component of the treatment system 100.

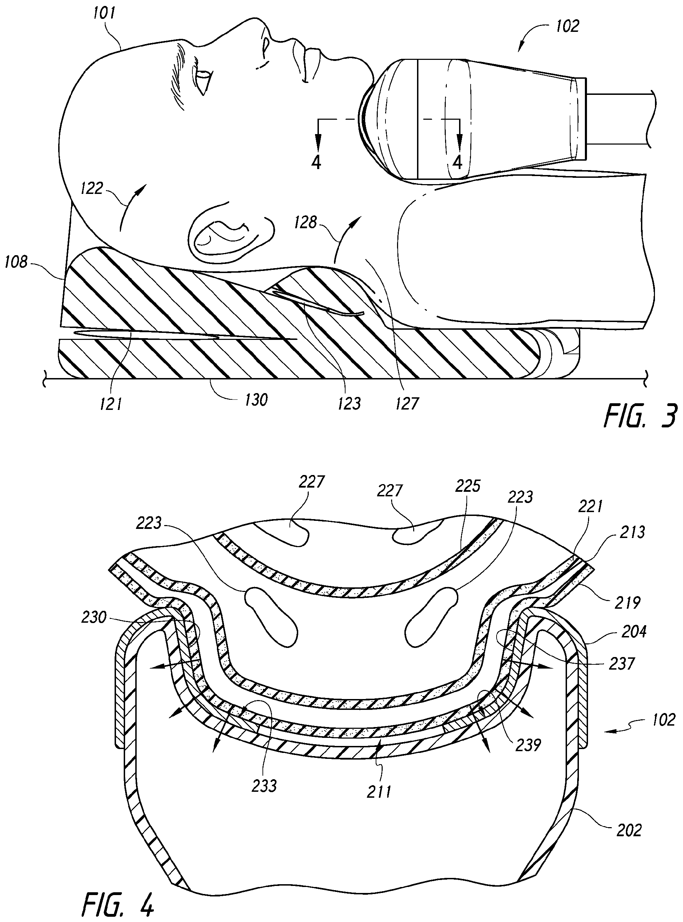

Figure 3 is a side view of the applicator 102 applied to the subject 101 while the pillow 130 (shown in cross section) supports the subject in accordance with embodiments of the technology. The pillow 130 can position the subject's body such that the submental region is at a suitable position for treatment by the applicator 102. An expandable member 121 of the head adjuster device 113 (Figure 1) can be expanded to tilt (indicated by arrow 122) the subject's head 109. An expandable member 123 of the neck adjuster device 115 (Figure 1) can be expanded such that the pillow 130 pushes against and moves the subject's neck 127 (indicated by arrow 128). The expandable members 121, 123 can be independently inflated/deflated any number of times in a treatment session.

### D. Applicators

Figure 4 is a cross-sectional view of the applicator 102 taken along line 4-4 of Figure 3. The applicator 102 can include an applicator unit 202 and a liner assembly 204. Tissue can be drawn into a tissue-receiving cavity 230 ("cavity 230") and against a patient-contact surface 237 of the liner assembly 204. The applicator unit 202 can extract heat from tissue 211 located in the tissue-receiving cavity 230. Heat (represented by arrows) from the tissue 211 can be conductively transferred through the liner assembly 204 to temperature-controlled heat-exchanging surfaces 239 of the applicator unit 202 such that heat flows across substantially all of the applicator/skin interface.

To effectively cool relatively shallow targeted submental tissue without adversely effecting deeper non-targeted tissue, the tissue 211 can be drawn against the bottom 233 of the relatively shallow tissue-receiving cavity 230. Subcutaneous lipid-rich cells in a subcutaneous layer 213 can be cooled an amount sufficient to be biologically effective in affecting (e.g., damaging and/or reducing) such lipid-rich cells without affecting non-target cells to the same or greater extent. In some procedures, platysma muscle 221, digastric muscle 223, mylohyoid muscle 225, geniohyoid muscle 227, and/or other non-targeted tissues can be generally unaffected by the treatment. In some procedures, adipose tissue in the subcutaneous layer 213 can be selectively cooled/heated without significantly affecting non-targeted tissue. Although the illustrated applicator 102 is positioned to treat mostly submental tissue, it can also be positioned to treat tissue at the submandibular region, neck region, or other target regions. Straps, harnesses, or other retaining apparatuses can secure the applicator 102 to the subject throughout therapy.

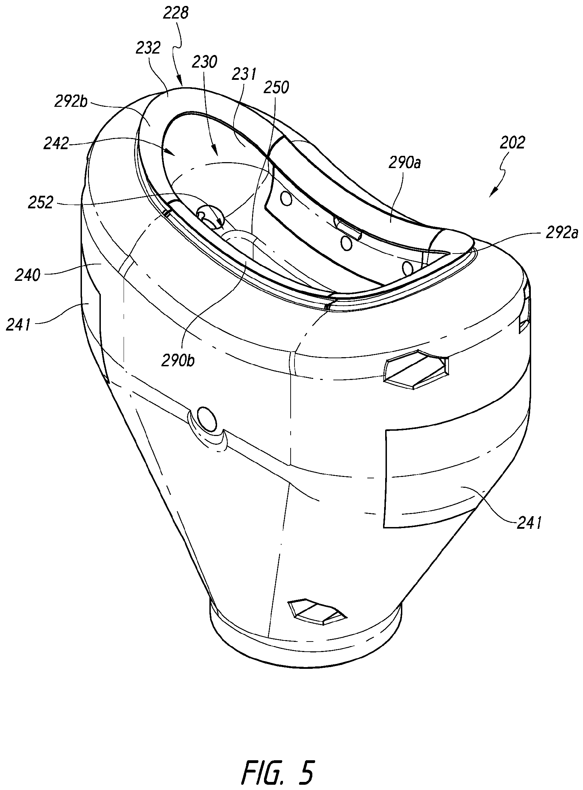

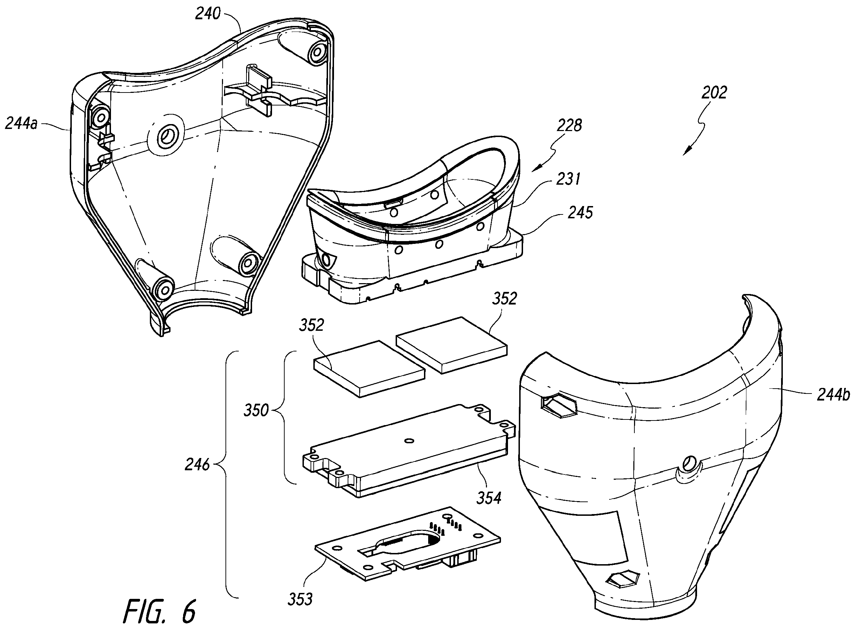

Figure 5 is an isometric view of the applicator unit 202 in accordance with embodiments of the technology. The applicator unit 202 can include a cup assembly 228 for cooling/heating tissue and a housing 240 for protecting the cup assembly 228. The cup assembly 228 can include a cup 231 and a contoured lip 232. The cup 231 can be contoured to accommodate tissue pulled into the cavity 230 and can serve as a heat sink to provide effective cooling/heating of tissue. The contoured lip 232 can define a mouth 242 of the cavity 230 and can sealingly engage, for example, a liner assembly (e.g., liner assembly 204 of Figure 3), the subject's skin (e.g., if the contoured lip 232 is placed directly against skin), a cryoprotectant gel pad, or other surface. The contoured lip 232 can include two spaced apart arcuate lip portions 290a, 290b and side lip portions 292a, 292b connecting the lip portions 290a, 290b. Fasteners 241 (e.g., hook or loop fastener) can be coupled to or part of the housing 240. Various features of the applicator unit 202 are discussed in detail in connection with Figures 6-9.

Figure 6 is an exploded isometric view of the applicator unit 202 in accordance with embodiments of the technology. In some embodiments, including the illustrated embodiment, the housing 240 includes two housing sections 244a, 244b that cooperate to surround internal components, but the housing 240 can have a wide range of multi-piece or one-piece constructions selected based on the configuration of cooling unit 246 and/or the cup assembly 228. The cooling unit 246 can be in thermal communication with a base 245 of the cup assembly 228. In a cooling mode, the cooling unit 246 cools the cup assembly 228, and in a heating mode, the cooling unit 246 heats the cup assembly 228.

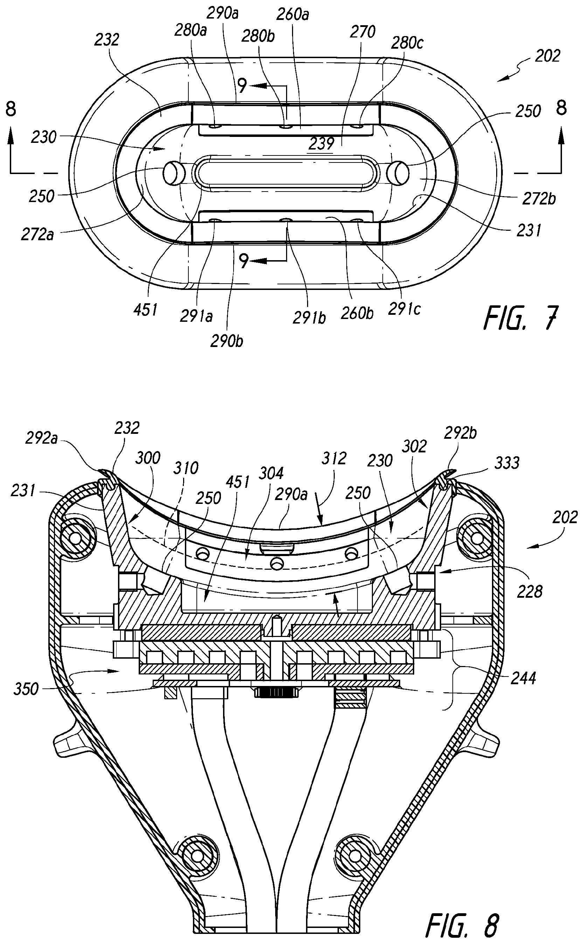

Figure 7 is a top view of the applicator unit 202 in accordance with embodiments of the technology. The cup 231 can include spaced apart sidewalls 260a, 260b, a bottom 270, and end portions 272a, 272b. The sidewalls 260a, 260b can be curved, flat, or combinations thereof and can extend between the end portions 272a, 272b. The bottom 270 can extend between the sidewalls 260a, 260b and can extend between the end portions 272a, 272b.

The cup 231 can be a thermally conductive cup made, in whole or in part, of a thermally conductive material for rapid cooling and/or heating to, for example, reduce treatment times and/or produce generally flat temperature profiles over the heat-exchanging surface 239 or a portion thereof. Because the subject's body heat can be rapidly conducted to the cup 231, the cooled skin can be kept at a generally flat temperature profile (e.g., ±3°C of a target temperature) even though regions of the skin, or underlying tissue, may experience different amounts of blood flow. The thermally conductive materials can include, without limitation, metal/metal alloys (e.g., stainless steel, copper alloys, etc.), pure metal (e.g., pure copper), or other rigid or flexible high heat transfer materials such as thermally conductive plastics. In some embodiments, the thermally conductive material at room temperature can have a thermal conductivity equal to or greater than about 13 W/(mK), 50 W/(mK), 100 W/(mK), 200 W/(mK), 300 W/(mK), 350 W/(mK), and ranges encompassing such thermal conductivities. In some embodiments, the cup 231 can have a multi-piece construction with different pieces made of different materials to provide different amounts of heat flow at different locations. In other embodiments, the cup 231 has a unitary construction and is made of a single material, such as metal. The surface 239 can be a smooth surface that extends continuously along at least most of the cavity 230. When tissue is drawn against the surface 239, the skin can be slightly stretched to reduce the thickness of the skin to increase heat transfer between target tissue and the surface 239. Thus, the mechanical properties, thermal properties, shape, and/or dimensions of the cup 231 can be selected based on, for example, target treatment temperatures and/or desired volume of tissue to be drawn into the cavity 230.

One or more vacuum ports 250 can be in fluid communication with the cavity 230. The number and locations of the vacuum ports 250 can be selected based on, for example, desired tissue draw, considerations of patient comfort, and the desired vacuum level. If the vacuum level is too low, tissue will not be drawn adequately (or at all) into the cavity 230. If the vacuum level is too high, undesirable discomfort to the patient and/or tissue damage could occur. The vacuum ports 250 can be positioned near the bottom of the cavity 230 to comfortably draw the tissue deep into the cavity 230.

Vacuum ports 280a, 280b, 280c (collectively, "vacuum ports 280") can be positioned along the sidewall 260a, and vacuum ports 291a, 291b, 291c (collectively, "vacuum ports 291") can be positioned along the sidewall 260b. The vacuum ports 280, 291 can be used to draw a liner assembly, cryoprotectant gel pad, and/or tissue against the respective sidewalls 260a, 260b. In other embodiments, adhesive (e.g., pressure-sensitive adhesive), snaps, or hook and loop type fasteners can be positioned at various locations along the surface 239 and can couple a liner assembly to the applicator unit 202. In yet other embodiments, a cinching device (not shown) can couple liner assemblies to the applicator unit 202.

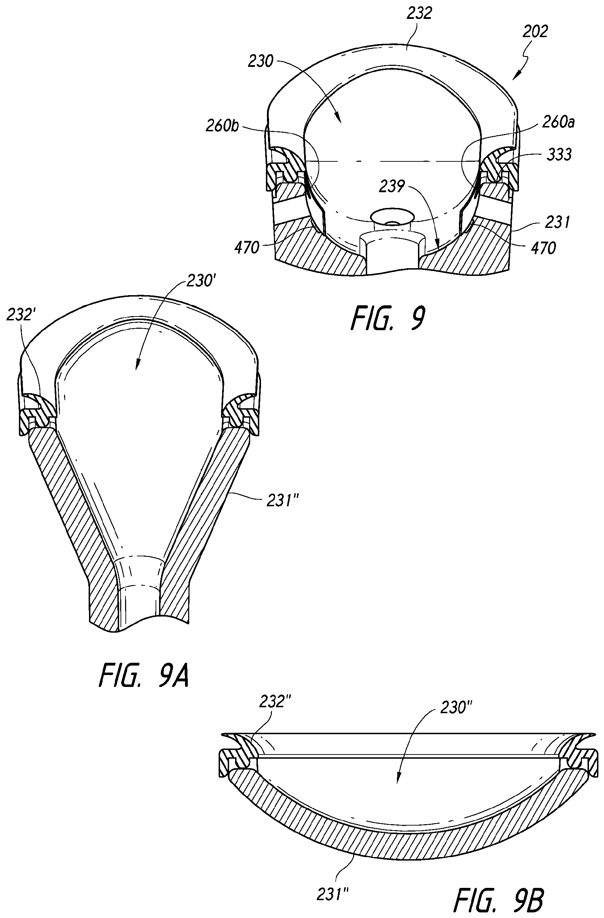

Figure 8 is a cross-sectional view of the applicator unit 202 taken along line 8-8 of Figure 7. Figure 9 is a cross-sectional view of the applicator unit 202 taken along line 9-9 of Figure 7. Referring now to Figure 8, cavity 230 can include a first end 300, a second end 302, and a central section 304 extending between the first and second ends 300, 302. The central section 304 can have a curved longitudinal axis 310 extending along a substantially circular path, an elliptical path, or other desired nonlinear or linear path. In some embodiments, the longitudinal axis 310 has a curvature generally equal to the curvature of at least one of the arcuate lip portions 290a, 290b (as viewed from the side). In other embodiments, the longitudinal axis 310 can have a curvature that is different than the curvature of one or both lip portions 290a, 290b and can be selected based on the shape of the subject's body.

The cavity 230 can have substantially uniform depth along most of curved longitudinal axis 310. Embodiments of the applicator unit 202 for treating submental tissue can have a maximum depth 312 equal to or less than about 0.5 cm, 2 cm, 2.5 cm, 3 cm, or 5 cm, for example. Embodiments of the applicator unit 202 for treating facial tissue can have a maximum depth 312 equal to or less than about 0.5 cm, 2 cm, or 3 cm, for example. The maximum depth 312 can be selected based on, for example, the volume of targeted tissue, characteristics of the targeted tissue, and/or desired level of patient comfort.

Figure 9 shows the sidewalls 260a, 260b splayed out to facilitate conformably drawing tissue into the tissue-receiving cavity 230. The positive draft angle of the sidewalls 260a, 260b can be increased or decreased to decrease or increase, respectively, the vacuum level needed to fill the cavity 230 with tissue. Referring to Figures 8 and 9 together, the bottom of the cavity 230 can define a curved longitudinal profile shape in a longitudinal direction (e.g., a direction parallel to the axis 310 in Figure 8), and the bottom of the cavity 230 can define a curved transverse profile shape in a transverse direction. In one embodiment, a radius of curvature of the longitudinal curve profile shape of Figure 8 can be greater than a radius of curvature of the transverse curved profile of Figure 9. The tissue-receiving cavities disclosed herein can have substantially U-shaped cross sections (see cavity 230 of Figure 9), V-shaped cross sections (see tissue-receiving cavity 230' of Figure 9A), or partially circular/elliptical cross-sections (see tissue-receiving cavity 230" of Figure 9B), as well as or other cross sections suitable for receiving tissue.

Figure 9 shows the contoured lip 232 connected to an upper edge 333 of the cup 231. The contoured lip 232 can be made, in whole or in part, of silicon, rubber, soft plastic, or other suitable highly compliant materials. The mechanical properties, thermal properties, shape, and/or dimensions of the contoured lip 232 can be selected based on, for example, whether the contoured lip 232 contacts a liner assembly, a surface of a cryoprotectant gel pad, or the subject's skin.

Sensors 470 can be coupled to the surface 239, embedded in the cup 231, or located at other suitable positions (e.g., carried by a film applied to the cup 231). The sensors 470 can be temperature sensors, such as thermistors, positioned to detect temperature changes associated with warm tissue being drawn into the cup 231. A control module (e.g., control module 106 of Figure 1) can interpret the detected temperature increase associated with skin contact and can monitor, for example, the depth of tissue draw and tissue contact based on the locations and amount of temperature increase. In some embodiments, the sensors 470 measure heat flux and/or pressure (e.g., contact pressure) with the skin of the patient. In yet further embodiments, the sensors 470 can be tissue impedance sensors or other sensors capable of detecting the presence and/or characteristics of tissue. Feedback from the sensors 470 can be collected in real-time and used in concert with treatment administration to efficaciously target specific tissue. The sensor measurements can also indicate other changes or anomalies that can occur during treatment administration. For example, an increase in temperature detected by the sensors 470 can indicate either a freezing event at the skin or movement of the applicator 102. An operator can inspect the subject's skin and/or applicator 102 in response to a detected increase in temperature. Methods and systems for collection of feedback data and monitoring of temperature measurements are described in commonly assigned U.S. Patent No. 8,285,390.

Referring again to Figure 6, the cooling unit 246 can be mounted directly to the cup assembly 228 and can include a thermal device 350 and a connection assembly 353. The thermal device 350 can include, without limitation, one or more thermoelectric elements (e.g., Peltier-type elements), fluid-cooled elements, heat-exchanging units, or combinations thereof. In some embodiments, the thermal device 350 includes thermoelectric elements 352 for cooling/heating the base 245 and a fluid-cooled element 354 for cooling/heating the thermoelectric elements 352. In a cooling mode, the fluid-cooled element 354 can cool the backside of the thermoelectric elements 352 to keep the thermoelectric elements 352 at or below a target temperature. In a heating mode, the fluid-cooled element 354 can heat the backside of the thermoelectric elements 352 to keep the thermoelectric elements 352 at or above a target temperature. Although the illustrated thermal device 350 has two thermoelectric elements 352, it can have any desired number of thermoelectric elements 352 at various locations about the cup 231. In other embodiments, the thermal device 350 has only fluid-cooled elements or only non-fluid cooled thermoelectric elements. The configurations and components of the thermal device 350 can be selected based on the desired power consumption and targeted temperatures. The connection assembly 353 can include circuitry, a circuit board, fittings (e.g., inlet ports, outlet ports, etc.), or the like. The cooling unit 246 can also be incorporated into part of the cup assembly 228. In such embodiments, the thermoelectric elements 352 can be embedded or otherwise disposed in the cup 231 to reduce the distance from the tissue to the thermoelectric elements 352.

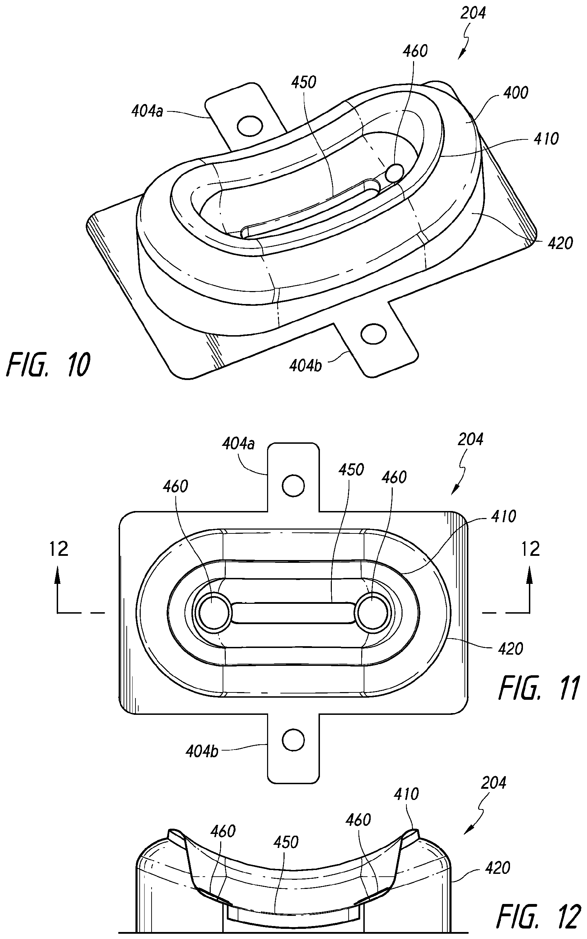

Figure 10 is an isometric view of the liner assembly 204 in accordance with one embodiment. Figure 11 is a top view of the liner assembly 204 of Figure 10. Figure 12 is a cross-sectional view of the liner assembly 204 taken along line 12-12 of Figure 11. When the liner assembly 204 is positioned on an applicator unit, the liner assembly 204 can provide a sanitary surface for contacting a patient and can also effectively transfer heat between the subject and the applicator unit. After treatment, the liner assembly 204 can be discarded or sanitized and reused.

The liner assembly 204 can include a cup liner 400 for overlaying the heat transfer surface of an applicator unit and attachment members 404a, 404b for securing the liner assembly 204 to the applicator unit. The cup liner 400 can include a lip portion 410 and a main body 420. When the applicator unit is inserted into the main body 420, the lip portion 410 can surround the mouth of a tissue receiving cavity and an elongated opening 450 can be aligned with a trench (see trench 451 of Figure 8) of the applicator unit, and openings 460 can be aligned with the vacuum ports of the applicator unit. In highly compliant embodiments, the liner assembly 204 can be made, in whole or in part, of rubber, soft plastic, or other compliant material.

Liner assemblies can also be a film, a sheet, a sleeve, or other component suitable for defining an interface surface to prevent direct contact between the applicator unit and the subject's skin to reduce the likelihood of cross-contamination between patients, minimize cleaning requirements, etc. Exemplary protective liners can be sheets, sleeves, or other components constructed from latex, rubber, nylon, Kevlar^{®}, or other substantially impermeable or semi-permeable material. Further details regarding a patient protection device may be found in U.S. Patent Publication No. 2008/0077201. A liner or protective sleeve may be positioned between the absorbent and the applicator to shield the applicator and to provide a sanitary barrier that is, in some embodiments, inexpensive and thus disposable.

### E. Treatment Methods

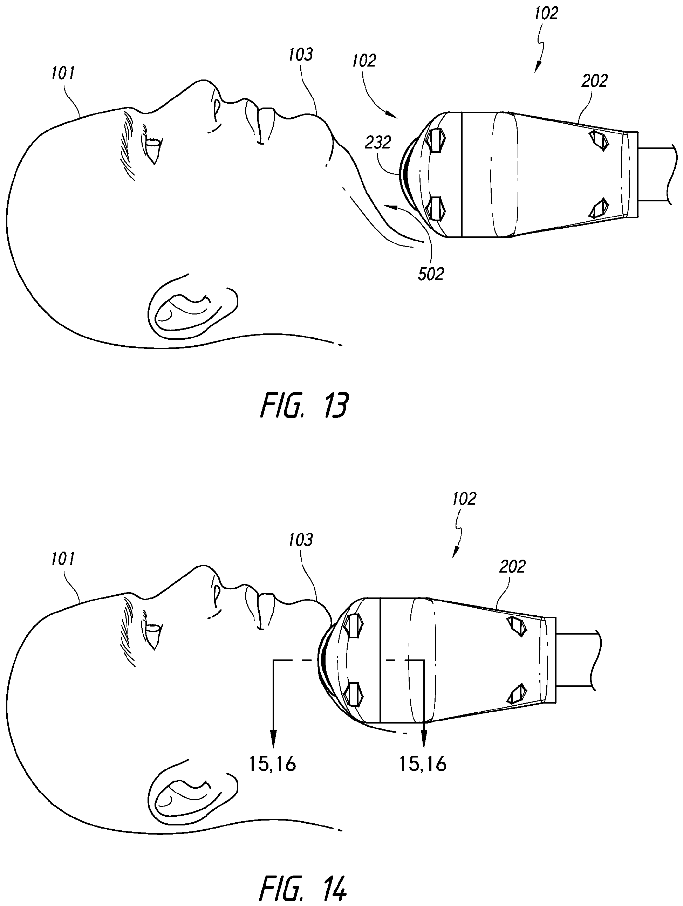

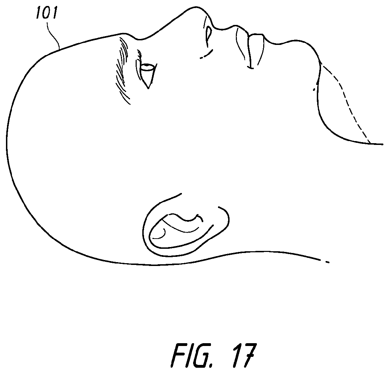

Figures 13-17 are a series of views of a method of performing cryotherapy in accordance with various embodiments of the present technology. Generally, targeted tissue can be drawn into the applicator 102 until the tissue is in thermal contact a region of the cup assembly 228 located at a bottom of the cavity 230. The cup assembly 228 can be cooled to extract heat from the tissue so as to cool/heat targeted tissue an amount sufficient to be biologically effective in damaging and/or reducing targeted cells. Figure 17 shows a pretreatment tissue profile of a double chin in phantom line and the post treatment tissue profile in solid line. Various details of operation are discussed in detail below.

Figure 13 shows the applicator 102 ready to be placed at a treatment site 502. In procedures for reducing a double chin, the applicator 102 can be aligned with and placed generally at the submental region (i.e., the submental triangle). Although the subject's head is shown at a generally horizontal orientation, the subject's head can be held at other orientations. For example, a pillow (e.g., pillow 130 of Figure 1) or other support device can be used to elevate, tilt, or otherwise position the subject's head, neck, shoulders, and/or other body parts. The applicator 102 can be placed against the subject such that it extends laterally across the submental triangle or a portion thereof. It will be appreciated that the applicator 102 can be placed at other locations along the patient's body and the orientation of the applicator 102 can be selected to facilitate a relatively close fit.

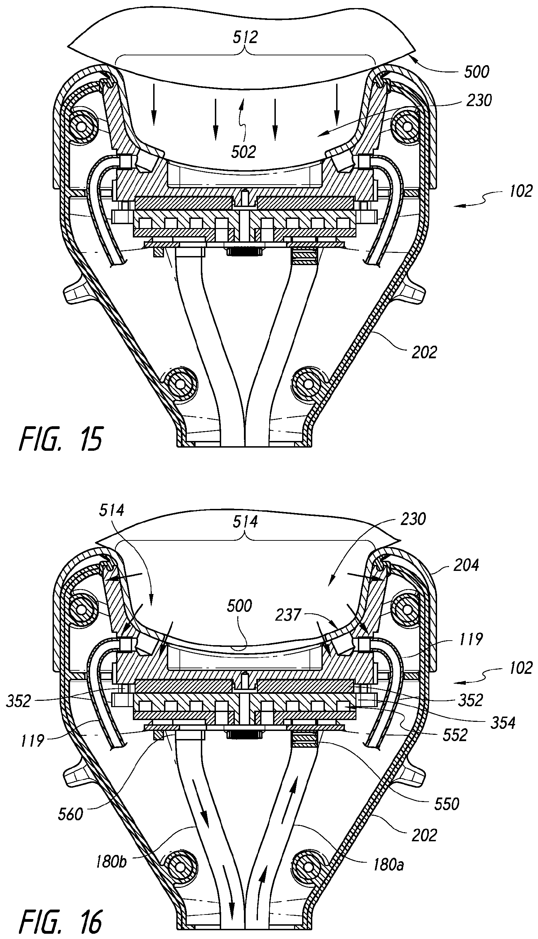

Figure 14 shows the applicator 102 placed against the subject's skin. Figure 15 is a cross-sectional view of the applicator 102 contacting the subject's skin 500 before drawing tissue. Figure 16 is a cross-sectional view of the applicator 102 after tissue has been drawn into the cavity 230. Although not shown in Figures 13-16 for ease of illustration, other elements, materials, components (e.g., gel pads, absorbents, etc.) can be located between the skin 500 and the applicator 102. U.S. Pub. No. 2007/0255362 and U.S. Patent Publication No. 2008/0077201 and U.S. App. No. 14/610,807 disclose components, materials (e.g., coupling gels, cryoprotectants, compositions, etc.), and elements (e.g., coupling devices, liners/protective sleeves, absorbents, etc.) that can be placed between the skin 500 and the applicator 102.

Referring to Figures 15 and 16, when a vacuum is applied, the skin 500 can be moved (indicated by arrows in Figure 15) towards the bottom of the cavity 230. The vacuum level can be selected to comfortably pull the tissue into contact with the desired area of the applicator 102, and the skin 500 and underlying tissue can be pulled away from the subject's body which can assist in cooling underlying tissue by, e.g., lengthening the distance between targeted subcutaneous fat and the muscle tissue. After a sufficient amount of tissue fills most or all of the cavity 230, the tissue is cooled/heated. Figure 16 shows mostly submental tissue located in the cavity 230. For example, substantially all the tissue 514 can be submental tissue to alter only the submental region. In other procedures, tissue at the submandibular region can be drawn into the cavity 230 to reduce, for example, jowl fat.

Because a target volume of fat may be relatively small and localized, the applicator 102 can provide well-defined margins of the treatment area. In some embodiments, the applicator 102 can conductively cool an area equal to or less than about 20 cm², 30 cm², or 40 cm² to avoid damaging non-targeted tissue (e.g., tissue adjacent to the submental region). In some embodiments, the patient-contact surface 237 can have a surface area equal to or less than about 20 cm², 30 cm², or 40 cm². An operator can have an array of applicators with different dimensions so that the operator can select an applicator to match a patient's anatomy.

The control module 106 (Figure 1) can automatically begin heating/cooling the tissue. In other embodiments, the control module 106 (Figure 1) can notify the operator that the applicator 102 is ready for treatment. The operator can inspect the applicator 102 and can begin treatment using the control module 106. Heat (represented by arrows in Figure 16) can be transferred from targeted tissue to the thermoelectric elements 352. Coolant can flow through an inlet port 550 connected to the fluid line 180a. The coolant can circulate through passages 552 to absorb heat from the thermoelectric elements 352 and can exit the passages 552 via the outlet port 560 connected to the fluid line 180b. The heated coolant can flow back to the control module 106 (Figure 1) for cooling.

In contrast to invasive procedures in which coolant is injected directly into targeted tissue, each of the sidewalls 260a, 260b and bottom 270 (Figure 7) can conductively cool tissue to produce a desired temperature in target tissue without bruising, pain, or other problems caused by injections and perfusion of injected fluid. For example, perfusion of injected fluid can affect the thermal characteristics of the treatment site and result in undesired temperature profiles. As such, the non-invasive conductive cooling provided by the applicator 102 can be more accurate than invasive procedures that rely on injecting fluids. The illustrated targeted tissue of Figure 16 can be cooled to a temperature range from about -20°C to about 10°C, from about 0°C to about 20°C, from about -15°C to about 5°C, from about -5°C to about 15°C, or from about -10°C to about 0°C. In one embodiment, the patient-contact surface 237 can be kept at a temperature less than about 5°C to extract heat from subcutaneous lipid-rich cells such that those cells are selectively reduced or damaged. Because non-lipid-rich cells usually can withstand colder temperatures better than lipid-rich cells, the subcutaneous lipid-rich cells can be injured selectively while maintaining the non-lipid-rich cells (e.g., non-lipid-rich cells in the dermis and epidermis).

Lines 119 of Figure 16 can provide sufficient vacuum to hold the skin 500 against the patient-contact surface 237. The tissue 514 can fill substantially the entire cavity 230. For example, the tissue 514 can occupy at least 70%, 80%, 90%, or 90% of the volume of the cavity 230 to avoid or minimize air pockets that may impair heat transfer. The restraint apparatus 107 of Figure 1 can be adjusted such that the applicator 102 applies sufficient pressure to reduce, limit, or eliminate blood flow to deeper tissue to improve cooling efficiency because blood circulation is one mechanism for maintaining a constant body temperature of about 37°C. Blood flow through the dermis and subcutaneous layer of the tissue is a heat source that counteracts the cooling of the targeted tissue (e.g., sub-dermal fat). If the blood flow is not reduced, cooling the subcutaneous tissues would require not only removing the specific heat of the tissues but also that of the blood circulating through the tissues. Thus, reducing or eliminating blood flow through the tissue 514 can improve the efficiency of cooling and avoid excessive heat loss from the dermis and epidermis.

It will be appreciated that while a region of the body has been cooled or heated to the target temperature, in actuality that region of the body may be close but not equal to the target temperature, e.g., because of the body's natural heating and cooling variations. Thus, although the applicator 102 may attempt to heat or cool the target tissue to the target temperature or to provide a target heat flux, the sensors 470 (Figure 9) may measure a sufficiently close temperature or heat flux. If the target temperature or heat flux has not been reached, operation of the cooling unit can be adjusted to change the heat flux to maintain the target temperature or "set-point" selectively to affect targeted tissue. When the prescribed segment duration expires, the next treatment profile segment can be performed.

Figure 17 shows subject after completing cryotherapy with the pretreatment tissue profile of a double chin (shown in phantom line) and the post treatment tissue profile without the double chin (shown in solid line). It may take a few days to a few weeks, or longer, for the adipocytes to break down and be absorbed. A significant decrease in fat thickness may occur gradually over 1-3 months following treatment. Additional treatments can be performed until a desired result is achieved. For example, one or more treatments can be performed to substantially reduce (e.g., visibly reduce) or eliminate a double chin.

The treatment procedure of Figures 13-17 can also involve use of cryoprotectant between the applicator 102 and the skin. The cryoprotectant can be a freezing point temperature depressant that may additionally include a thickening agent, a pH buffer, a humectant, a surfactant, and/or other additives. The temperature depressant may include, for example, polypropylene glycol (PPG), polyethylene glycol (PEG), dimethyl sulfoxide (DMSO), or other suitable alcohol compounds. In a particular embodiment, a cryoprotectant may include about 30% polypropylene glycol, about 30% glycerin (a humectant), and about 40% ethanol. In another embodiment, a cryoprotectant may include about 40% propylene glycol, about 0.8% hydroxyethylcellulose (a thickening agent), and about 59.2% water. In a further embodiment, a cryoprotectant may include about 50% polypropylene glycol, about 40% glycerin, and about 10% ethanol. Other cryoprotectants or agents can also be used and can be carried by a cotton pad or other element. U.S. App. No. 14/610,807 is incorporated by reference in its entirety and discloses various compositions that can be used as cryoprotectants.

### F. Applicator Units

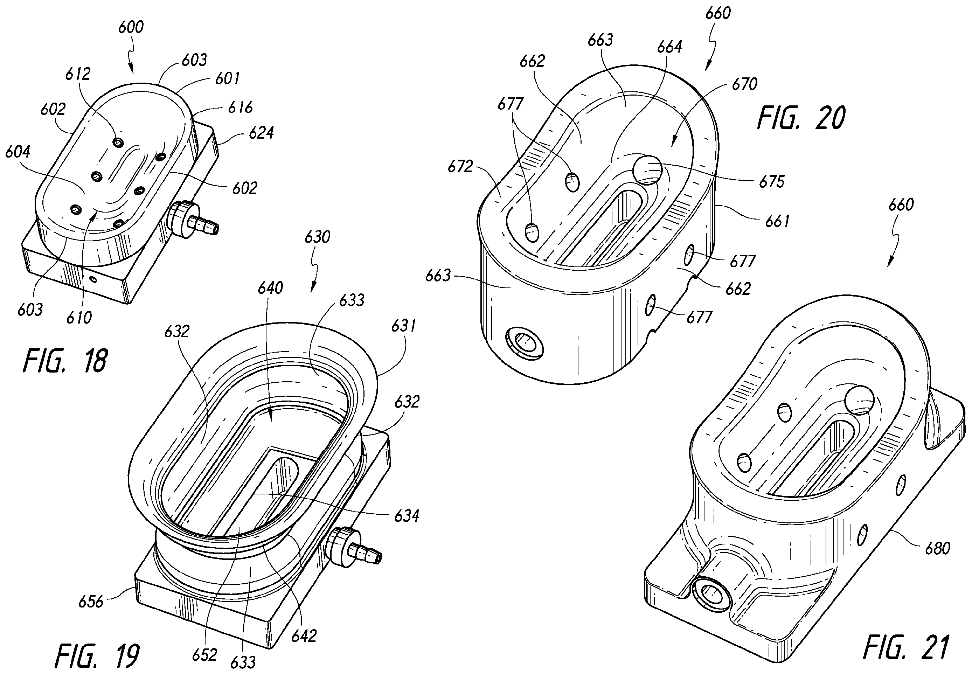

Figures 18-22 are isometric views of applicators in accordance with embodiments of the present technology. The description of the applicator 102 (Figures 1-17) applies equally to the applicators of Figures 18-21 unless indicated otherwise. Figure 18 shows an applicator 600 that includes a cup 601 defining a tissue-receiving cavity 610. The cup 601 has sidewalls 602, end portions 603, and a bottom 604. A thermally conductive edge 616 (e.g., a rounded edge or a blunt edge) can be made of metal or other thermally conductive material capable of cooling/heating margins of the treatment site. An array of vacuum ports 612 (one labeled in Figure 18) are in fluid communication with the tissue-receiving cavity 610. A base 624 can be in thermal communication with the cup 601 and can include, without limitation, one or more thermal elements, controllers, or the like.

Figure 19 shows an applicator 630 that includes a cup 631 and a base 656. The cup 631 has sidewalls 632, end portions 633, and a bottom 634 and defines a tissue-receiving cavity 640. A lip portion 642 is coupled to the cup 631 and flares outwardly. The cavity 640 (as viewed from above) can have an elongate shape (e.g., generally elliptical shape, rounded rectangle shape, etc.), a circular shape, or other suitable shape for receiving tissue. A vacuum port 652 is in fluid communication with the tissue-receiving cavity 640.

Figure 20 shows an applicator 660 that includes a cup 661 with sidewalls 662, end portions 663, and a bottom 664 and can define a tissue-receiving cavity 670. A lip portion 672 can be a bladder seal or other sealing member. A vacuum port 675 can provide a vacuum for drawing the submental tissue into the cup 661, and vacuum ports 677 can provide a vacuum for drawing a liner assembly or skin against the cup 661. Figure 21 shows the applicator 660 with a base 680 that serves as a heat spreader to increase heat flows.

Exemplary components and features that can be incorporated into the applicators disclosed herein are described in, e.g., commonly assigned U.S. Patent No. 7,854,754 and U.S. Patent Publication Nos. 2008/0077201, 2008/0077211, 2008/0287839, 2011/0238050 and 2011/0238051. The patient protection devices (e.g., liners or liner assemblies) may also include or incorporate various storage, computing, and communications devices, such as a radio frequency identification (RFID) component, allowing for example, use to be monitored and/or metered. Additionally, restraint apparatuses or components disclosed herein can be used to perform the method discussed in connection with Figures 13 - 16. For example, the restraint apparatus 107 can be used to hold the applicators disclosed herein to perform the cryotherapy of Figures 13 - 16.

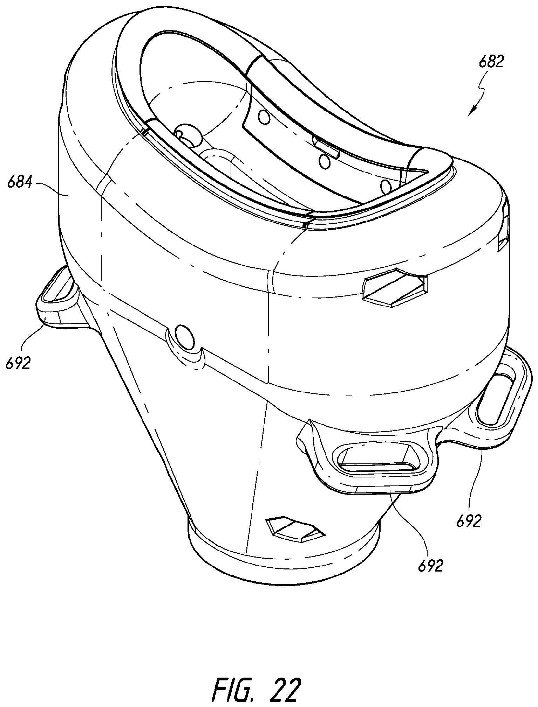

Figure 22 is an isometric view of an applicator 682 in accordance with embodiments of the technology. The applicator 682 is generally similar to the applicator 102 discussed in connection with Figures 1-17. The applicator 682 of Figure 22 includes a housing 684 with coupling features in the form of loops 692 configured to receive restraints, such as flexible straps, belts, etc.

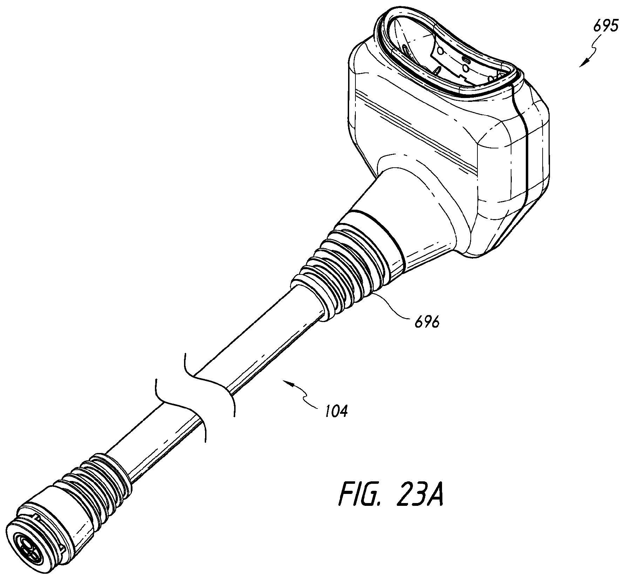

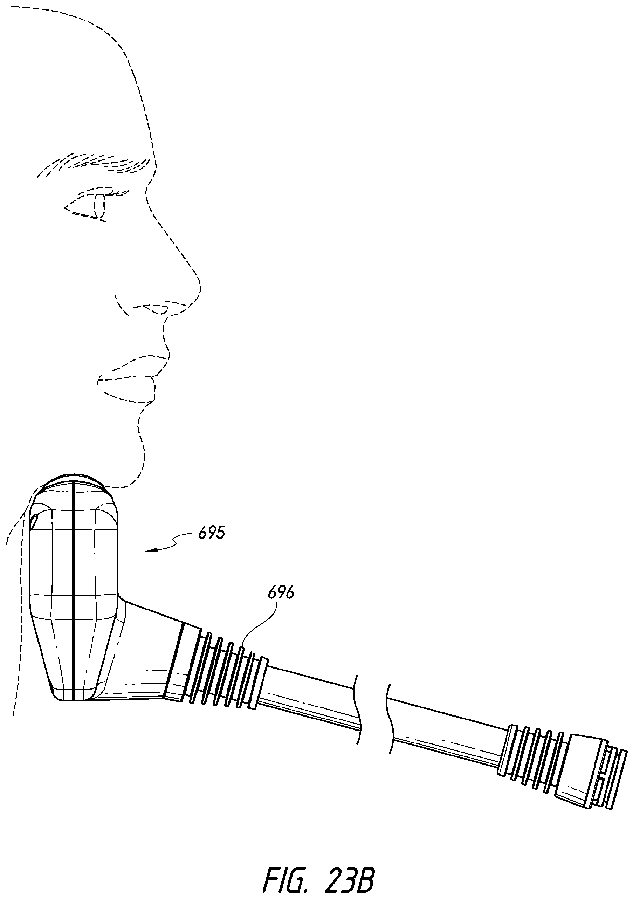

Figure 23A is an isometric view of an applicator 695 suitable for use with treatment systems disclosed herein. The applicator 695 can be generally similar to the applicators discussed in connection with Figures 1-23. The applicator 695 is connected to a connector 104 via a flexible joint 696. Figure 23B shows the flexible joint 696 extending from a side of the applicator 695 or from any other suitable location along the applicator 695.

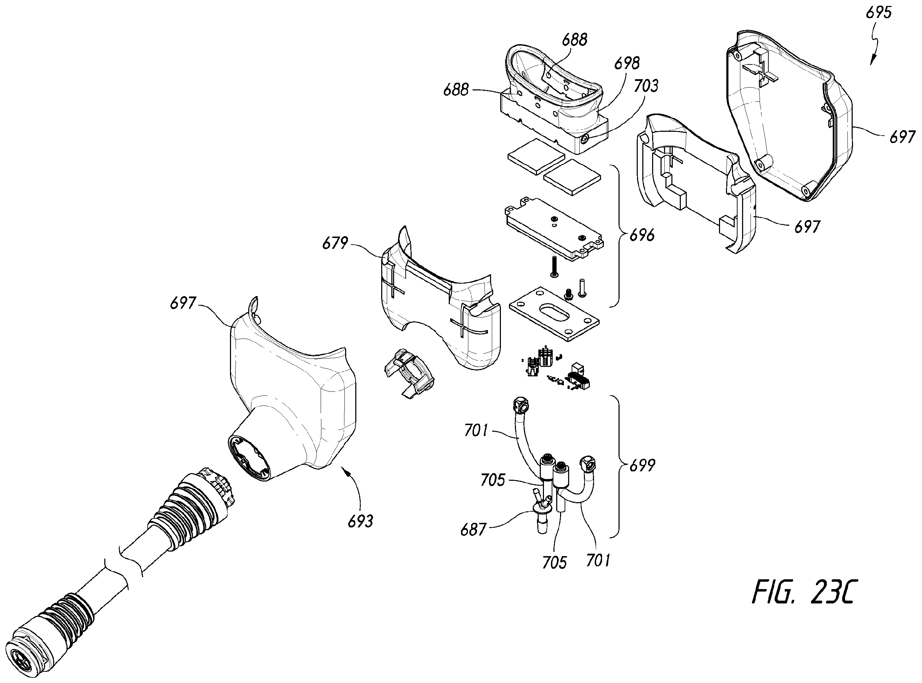

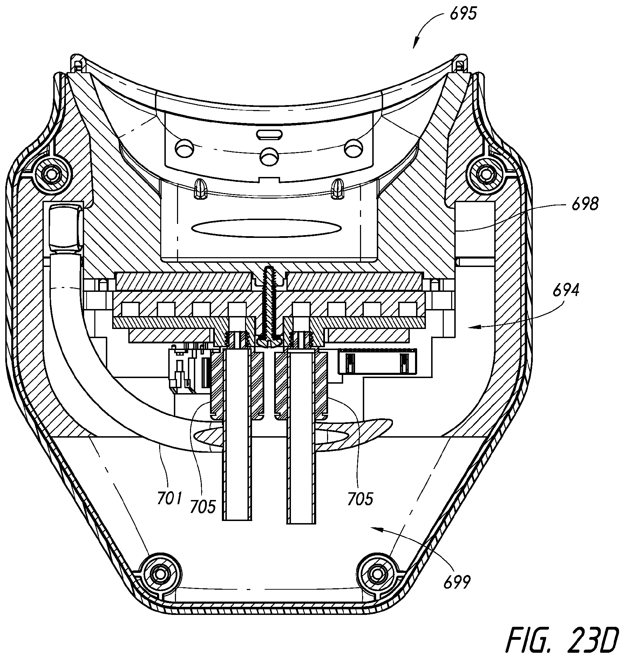

Figure 23C is an exploded isometric view of the applicator 695 in accordance with embodiments of the technology, and Figure 23D is a cross-sectional view of the applicator of Figure 23A. Referring to these figures, a housing 693 of the applicator 695 can include multiple housing sections 697 that cooperate to surround and protect internal components, such as a cooling unit 694. The cooling unit 694 can heat or cool a conductive cup 698. A manifold system 699 can include lines 701 in fluid communication with ports 703 (one identified in Figure 23C) of the cup 698. The manifold system 699 can also include coolant lines 705 (Figure 23D) that provide coolant to and take away coolant from the cooling unit 694. The applicator 695 can have other components, including liner assemblies, sensors, manifolds, vibrators, massage devices, or combinations thereof. Additionally, the manifold system 699 can include vacuum lines 687 that can be fluid communication with vacuum ports 688 (two identified in Figure 23C) of the cup 698. The vacuum lines 687 can be used to draw a vacuum to hold a liner assembly against the conductive cup 698.

Although noninvasive applicators are illustrated and discussed with respect to Figures 1-23D, minimally invasive applicators may also be employed. As an example, a cryoprobe, an electrode, an injector (e.g., a needle), and/or other invasive component may be incorporated into the applicators disclosed herein and can be inserted directly into the targeted tissue (e.g., subcutaneous adipose tissue) to cool, freeze, or otherwise thermally process targeted tissue. Treatment systems and applicators disclosed herein can also include elements (e.g., electrodes, vibrators, etc.) for delivering energy, such as radiofrequency energy, ultrasound energy (e.g., low frequency ultrasound, high frequency ultrasound, etc.), mechanical massage, and/or electric fields. The energy can be selected to affect treatment by, for example, heating tissue. Additionally or alternatively, energy can be used to affect the crystal formation in non-targeted tissues while allowing cooling of the targeted tissue. In non-targeted cells or structures, non-thermal energy parameters may be selected to reduce ice crystal size and/or length, reduce freezing lethality, or the like. In targeted cells or structures, non-thermal energy parameters may be selected to enhance crystal nucleation. Thus, energy can be selectively applied to control therapy. The treatment systems disclosed herein may be used with a substance that may provide a thermal coupling between the subject's skin and the thermal element(s) to improve heat transfer therebetween. The substance may be a fluid, e.g., a liquid, a gel, or a paste, which may be hygroscopic, thermally conductive, and biocompatible.

### G. Restraint Systems

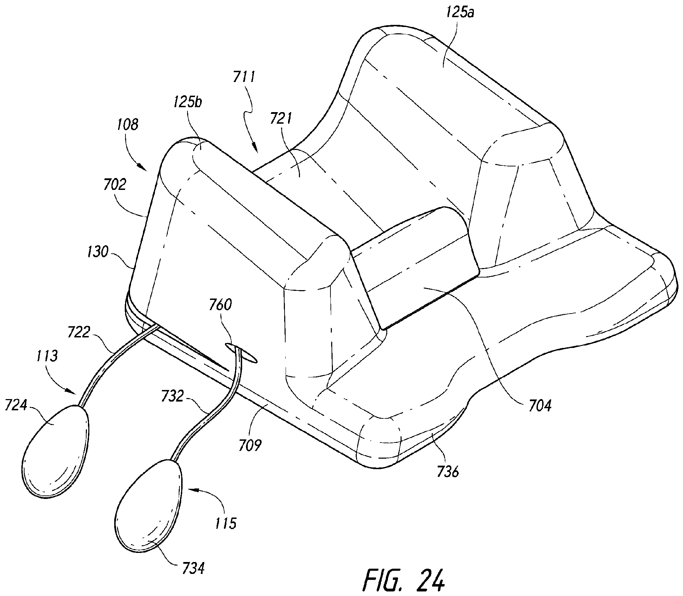

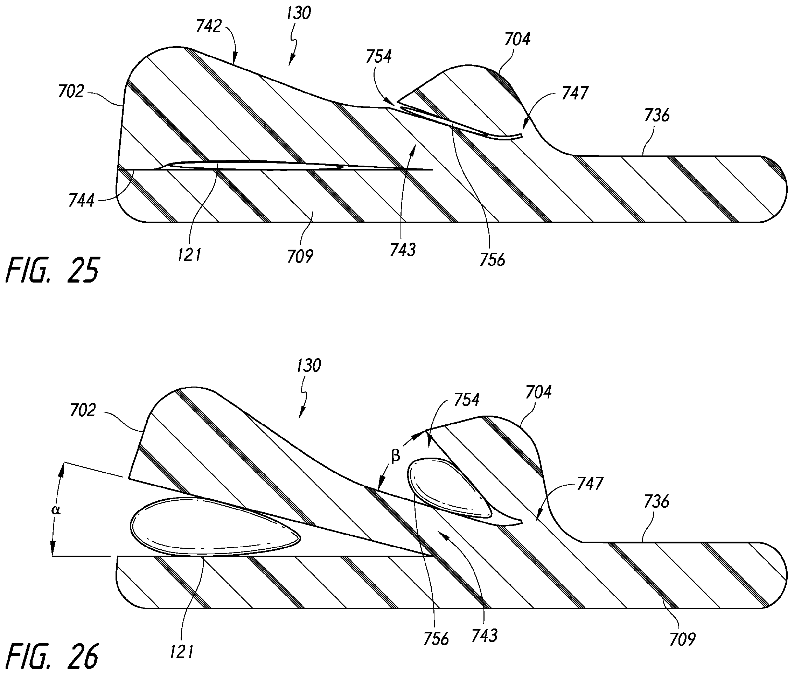

Figure 24 is an isometric view of the head support assembly 108 in accordance with embodiments of the present technology. The pillow 130 can include a deployable head cradle portion 702, a neck support portion 704, and a shoulder support portion 736. The head cradle portion 702 can include the vertical side portions 125a, 125b and a central region 721 therebetween. The side portions 125a, 125b are positioned to contact opposite sides of a subject's head located in a concave head-receiving region 711. Movement of the head cradle portion 702 and the neck support portion 704 for positioning the patient's body is discussed in connection with Figures 25 and 26.

The head adjuster device 113 can include a pressurization device in the form of a pump 724 and a conduit 722. The conduit 722 fluidically couples the pump 724 to an expandable member (not shown) positioned within the pillow 130. For example, the expandable member can be positioned between the head cradle portion 702 and the base 709. The pump 724 can be manually pumped to move the head cradle portion 702 to achieve desired tilt of the subject's head. The neck adjuster device 115 includes a pressurization device in the form of a pump 734 and a conduit 732. The conduit 732 can extend through the side portion 125b and to an expandable member located generally underneath the neck support portion 704. The pump 734 can be manually pumped to move the neck support portion 704 to achieve desired neck tilt of the subject. In various embodiments, the adjuster devices disclosed herein can include, without limitation, one or more motorized pumps, valves, pressure regulators, pneumatic drive devices, mechanical drive devices, or other suitable components.

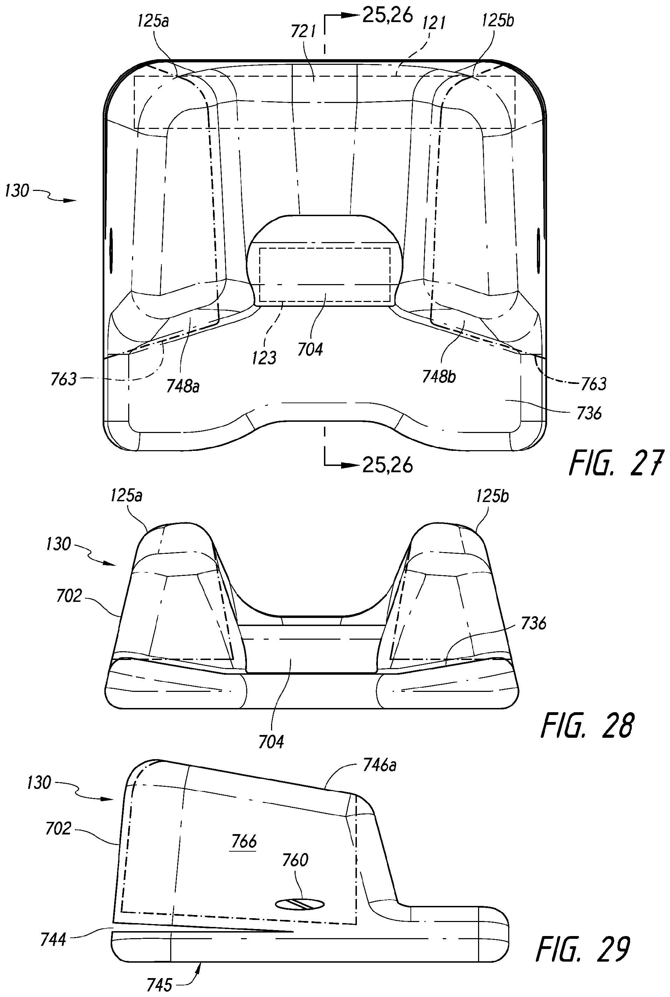

Figure 25 is a cross-sectional view of the pillow 130 taken along line 25-25 of Figure 27 when the pillow 130 is in an undeployed lowered configuration. Figure 26 is a cross-sectional view of the pillow 130 taken along line 26-26 of Figure 27 when the pillow 130 is in a deployed raised configuration. Referring now to Figure 25, the pillow 130 includes an expandable opening 744 positioned generally under a region of a head-support surface 742 of the head cradle portion 702. A deployable member 121 can be positioned in the expandable opening 744 and can be deployed (e.g., expanded, inflated, etc.) to move the head cradle portion 702. A flexible region or joint 743 can connect the head cradle portion 702 to the base 709. In some embodiments, the head cradle portion 702 can be rotated an angle ? when the expandable member 121 moves from an unexpanded configuration (Figure 25) to an expanded configuration (Figure 26). The angle ? can be greater than or equal to about 5 degrees, about 10 degrees, about 20 degrees, about 30 degrees, about 40 degrees, about 50 degrees, or about 60 degrees to rotate the support surface 742 a corresponding angle. The amount of movement of the head cradle portion 702 can be selected based on the desired amount of head tilt.

Figure 25 shows the neck support portion 704 positioned generally between the head cradle portion 702 and the shoulder support region 736. When the subject's head is supported by the head cradle portion 702, the neck support portion 704 is located under the subject's neck. A flexible portion or joint 747 can connect the neck support portion 704 to the base 709. An expandable opening 754 is located under the neck support portion 704. In some embodiments, an expandable member 123 can be inflated to push the neck support portion 704 upwardly to define an angle ? that is greater than or equal to about 5 degrees, about 10 degrees, about 15 degrees, about 20 degrees, about 25 degrees, about 30 degrees, about 35 degrees, about 40 degrees, or about 45 degrees to rotate the neck support portion 704 a corresponding angle. The amount of movement of the neck support portion 704 can be selected based on the desired support for the subject's neck.

The pillow 130 can include other types of movable features, such as movable panels (e.g., rotatable panels, linearly movable panels, etc.) or other features capable of being moved (e.g., translated, rotated, or both) to support, move, and/or otherwise interact with the subject's body. By way of example, the side portions 125 (Figure 24) can include surfaces or features that move inward firmly to hold the patient's head. The number, locations, and properties (e.g., cushioning properties, breathability, etc.) of the movable features can be selected based on, for example, desired patient comfort, body positioning, and/or treatment parameters.

Figure 27 is a top view of the pillow 130. The neck support portion 704 extends between shoulder-engagement ends 748a, 748b of the side portions 125a, 125b, respectively. The shoulder support region 736 is positioned to support the subject's shoulders when the shoulder-engagement ends 748a, 748b bear against the subject's right and left shoulders, respectively. The side portions 125a, 125b can include fasteners or other components for coupling to restraints (e.g., restraints 111a, 111b of Figure 1). In some embodiments, the side portions 125a, 125b include hook or loop fasteners 763 (illustrated in dash-dot lines) for coupling to loop or hook fasteners of the restraints 111. For example, the fastener 763 can be sections of hook Velcro^{®} closure. In other embodiments, the fastener 763 can include, without limitation, one or more snaps, buttons, ties, or other attachment features. Other regions of the pillow 130 can be made of breathable material and can have one-way or two-way stretchability.

Figure 28 is a front view of the pillow 130 with the head cradle portion 702 having a substantially U-shaped profile (including V-shaped). The head cradle portion 702 can also have a semi-circular shape profile or other suitable shape for accommodating the subject's head. The heights of the side portions 125a, 125b can be selected such that the side portions 125a, 125b extend upwardly along opposite sides of the subject's head sufficient distances to reduce or limit side-to-side rotation of the subject's head.

Figure 29 is a side view of the pillow 130. The expandable opening 744 can be a slot or a slit extending inwardly and generally parallel to a bottom surface 745 of the pillow 130. An access feature 760 in the form of a through-hole extends from an exterior surface 766 of the side portion 125a to the expandable opening 754 (Figures 25 and 26). The bottom surface 745 can comprise non-skid material for inhibiting movement of the pillow 130 along a support surface.

The pillow 130 can be made, in whole or in part, of a compressible material, including without limitation open-cell foam, closed-cell foam, or other compliant material. In some embodiments, the pillow 130 can be made of open-cell polyurethane foam. In some embodiments, the pillow 130 can include a cover for surrounding the foam main body. The cover can be removed and washed to provide a clean surface, and the cover can include fasteners (e.g., loop fastener, snaps, etc.) for coupling to restraints or other components.

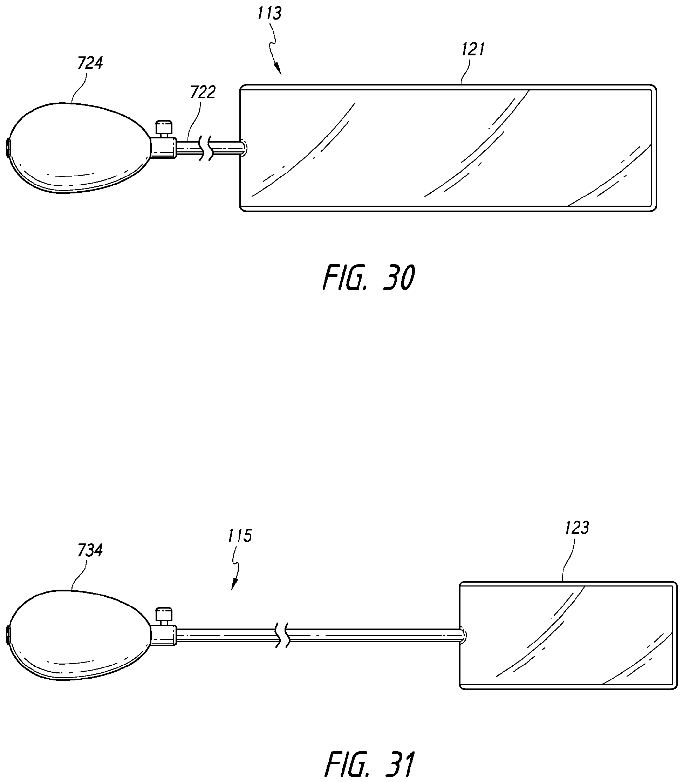

Figure 30 is a top view of the head adjuster device 113. The pump 724 can be a bulb pump, a squeeze pump, or other manual pump and may include a button for releasing air. In other embodiments, the pump 724 is a motorized pump. The conduit 722 can be flexible tubing that fluidically couples the pump 724 to the expandable member 121. The expandable member 121 can comprise, in whole or in part, urethane, silicon, rubber, or other suitable material. Referring now to Figure 27, the expandable member 121 (shown in phantom line) can be an inflatable bladder that extends across most of the width of the pillow 130.

Figure 31 is a top view of the neck adjuster device 115 generally similar to the head adjuster device 113 of Figure 30 except as detailed below. The expandable member 123 can comprise urethane, silicon, rubber, or other suitable material and can be dimensioned to be located under the neck support portion 704. Referring now to Figure 27, the expandable member 123 (shown in phantom line) is located generally between the side portions 125.

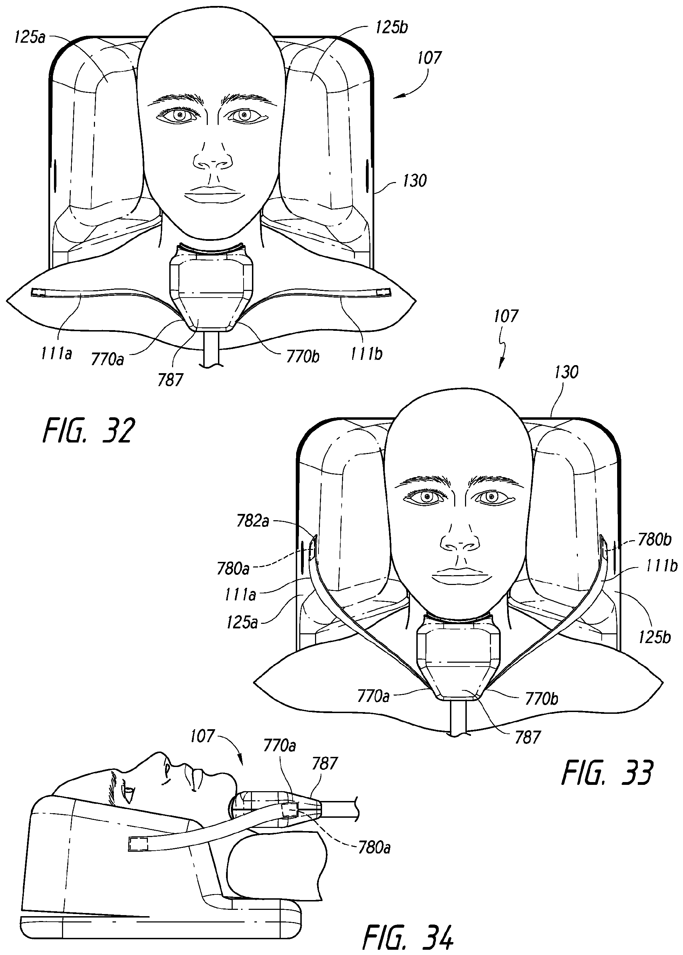

Figures 32-34 are a series of views of a method of performing cryotherapy using the restraint apparatus 107 in accordance with various embodiments of the present technology. Generally, the subject's head 109 can be positioned in the head cradle portion 702. An applicator 787 can then be aligned with the treatment site. The restraints 111a, 111b can be coupled to the side portions 125a, 125b and tensioned to pull the applicator 787 against the subject's submental region. After completing the treatment session, the restraints 111a, 111b can be detached from the respective side portions 125a, 125b to release the subject. Various details of operation are discussed in detail below.

Figure 32 is a top view of the pillow 130 supporting the subject's head and restraints 111a, 111b ready to be coupled to the pillow. The restraints 111a, 111b can be straps permanently or detachably coupled to an applicator 787. For example, ends 770a, 770b of the respective restraints 111a, 111b can include hook or loop fasteners, snaps, ties, and/or other features for coupling to the applicator 787. In other embodiments, the restraints 111a, 111b can be part of a harness system with a harness body holding the applicator 787. The number, lengths, and configurations of the restraints 111a, 111b can be selected based on the location of the treatment site, desired force for holding the applicator 787, or other treatment parameters.

Figure 33 is a top view of the restraint apparatus 107 holding the applicator 787 in thermal contact with the subject after fasteners 780a, 780b (illustrated in phantom line) of the restraints 111a, 111b have been applied to the pillow 130. The fasteners 780a, 780b can be loop fasteners located at or proximate to respective restraint ends 782a, 782b. Tensioning of the restraints 111, illustrated in a V arrangement, can be adjusted to inhibit or limit side-to-side movement of the applicator 787 and to stabilize the applicator 787 even if the subject's head moves slightly. During a single treatment session, the ends 782a, 782b can be coupled at various locations along the pillow 130 at different times, thus providing treatment flexibility.

Figure 34 is a left side view of the restraint apparatus 107 holding the applicator 787 in thermal contact with the subject. Referring to Figures 33 and 34, the restraint ends 770a, 770b can be permanently or detachably coupled to the applicator 787. For example, the restraint end 770a can include a fastener 787a (e.g., a loop fastener shown in phantom line in Figure 34) coupled to hook fastener of the applicator 787. The restraint ends 770 can be repositioned any number of times along the applicator 787. In other embodiments, the restraint ends 770 can be integrated into or part of the applicator 787, which can be similar or identical to the any of the applicators disclosed herein. The connection between the restraints 111 and the applicator 787 can be selected based on the design of the applicator.

### H. Computing Environments

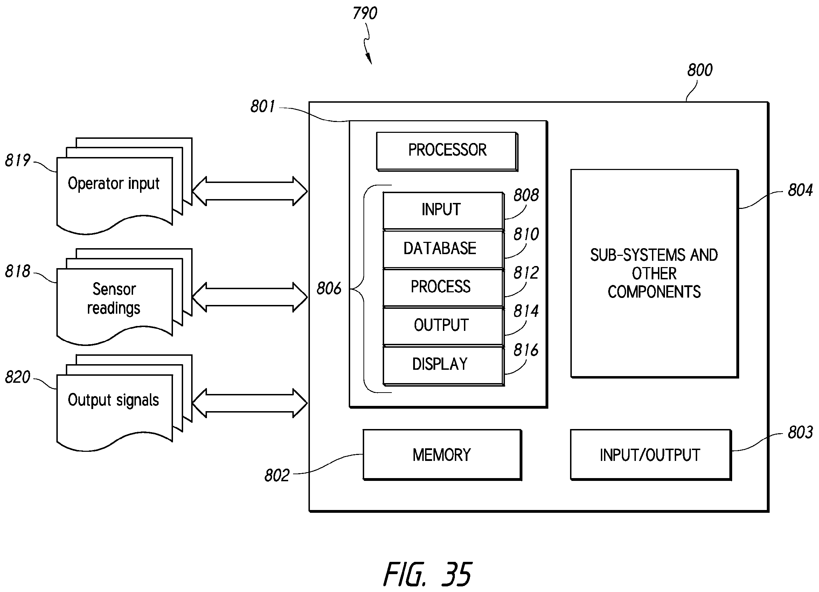

Figure 35 is a schematic block diagram illustrating subcomponents of a controller in accordance with an embodiment of the disclosure. The controller can be part of the control module 106 (Figure 1). For example, the controller 790 can be the controller 114 of Figure 1 or can be incorporated into the applicators or other components disclosed herein. The controller 790 can include a computing device 800 having a processor 801, a memory 802, input/output devices 803, and/or subsystems and other components 804. The computing device 800 can perform any of a wide variety of computing processing, storage, sensing, imaging, and/or other functions. Components of the computing device 800 may be housed in a single unit or distributed over multiple, interconnected units (e.g., though a communications network). The components of the computing device 800 can accordingly include local and/or remote memory storage devices and any of a wide variety of computer-readable media.

As illustrated in Figure 35, the processor 801 can include a plurality of functional modules 806, such as software modules, for execution by the processor 801. The various implementations of source code (i.e., in a conventional programming language) can be stored on a computer-readable storage medium or can be embodied on a transmission medium in a carrier wave. The modules 806 of the processor can include an input module 808, a database module 810, a process module 812, an output module 814, and, optionally, a display module 816.

In operation, the input module 808 accepts an operator input 819 via the one or more input devices, and communicates the accepted information or selections to other components for further processing. The database module 810 organizes records, including patient records, treatment data sets, treatment profiles and operating records and other operator activities, and facilitates storing and retrieving of these records to and from a data storage device (e.g., internal memory 802, an external database, etc.). Any type of database organization can be utilized, including a flat file system, hierarchical database, relational database, distributed database, etc.

In the illustrated example, the process module 812 can generate control variables based on sensor readings 818 from sensors and/or other data sources, and the output module 814 can communicate operator input to external computing devices and control variables to the controller. The display module 816 can be configured to convert and transmit processing parameters, sensor readings 818, output signals 820, input data, treatment profiles and prescribed operational parameters through one or more connected display devices, such as a display screen 118 (Figure 1), printer, speaker system, etc.

In various embodiments, the processor 801 can be a standard central processing unit or a secure processor. Secure processors can be special-purpose processors (e.g., reduced instruction set processor) that can withstand sophisticated attacks that attempt to extract data or programming logic. The secure processors may not have debugging pins that enable an external debugger to monitor the secure processor's execution or registers. In other embodiments, the system may employ a secure field programmable gate array, a smartcard, or other secure devices.

The memory 802 can be standard memory, secure memory, or a combination of both memory types. By employing a secure processor and/or secure memory, the system can ensure that data and instructions are both highly secure and sensitive operations such as decryption are shielded from observation. In various embodiments, the memory 802 can be flash memory, secure serial EEPROM, secure field programmable gate array, or secure application-specific integrated circuit. The memory 802 can store instructions for causing the applicators to cool/heat tissue, pressurization devices to draw a vacuum, or other acts disclosed herein. In one embodiment, the memory 802 stores instructions executable by the controller 790 for the thermal device to sufficiently cool conductive cups disclosed herein such that submental vacuum applicators non-invasively cool the subcutaneous lipid-rich cells to a desired temperature, such as a temperature less than about 0°C.

The input/output device 118 can include, without limitation, a touchscreen, a keyboard, a mouse, a stylus, a push button, a switch, a potentiometer, a scanner, an audio component such as a microphone, or any other device suitable for accepting user input and can also include one or more video monitor, a medium reader, an audio device such as a speaker, any combination thereof, and any other device or devices suitable for providing user feedback. For example, if an applicator moves an undesirable amount during a treatment session, the input/output device 803 can alert the subject and/or operator via an audible alarm. The input/output device 118 can be a touch screen that functions as both an input device and an output device. The control panel can include visual indicator devices or controls (e.g., indicator lights, numerical displays, etc.) and/or audio indicator devices or controls. The control panel may be a component separate from the input/output device 118 and/or output device 120, may be integrated applicators, may be partially integrated with one or more of the devices, may be in another location, and so on. In alternative embodiments, the controller 114 can be contained in, attached to, or integrated with the applicators. Further details with respect to components and/or operation of applicators, control modules (e.g., treatment units), and other components may be found in commonly-assigned U.S. Patent Publication No. 2008/0287839.

The controller 790 can include any processor, Programmable Logic Controller, Distributed Control System, secure processor, and the like. A secure processor can be implemented as an integrated circuit with access-controlled physical interfaces; tamper resistant containment; means of detecting and responding to physical tampering; secure storage; and shielded execution of computer-executable instructions. Some secure processors also provide cryptographic accelerator circuitry. Suitable computing environments and other computing devices and user interfaces are described in commonly assigned U.S. Patent No. 8,275,442, entitled "TREATMENT PLANNING SYSTEMS AND METHODS FOR BODY CONTOURING APPLICATIONS," which is incorporated herein in its entirety by reference.

### I. Conclusion

Various embodiments of the technology are described above. It will be appreciated that details set forth above are provided to describe the embodiments in a manner sufficient to enable a person skilled in the relevant art to make and use the disclosed embodiments. Several of the details and advantages, however, may not be necessary to practice some embodiments. Additionally, some well-known structures or functions may not be shown or described in detail, so as to avoid unnecessarily obscuring the relevant description of the various embodiments. Although some embodiments may be within the scope of the technology, they may not be described in detail with respect to the Figures. Furthermore, features, structures, or characteristics of various embodiments may be combined in any suitable manner. Moreover, one skilled in the art will recognize that there are a number of other technologies that could be used to perform functions similar to those described above. While processes or acts are presented in a given order, alternative embodiments may perform the processes or acts in a different order, and some processes or acts may be modified, deleted, and/or moved. The headings provided herein are for convenience only and do not interpret the scope or meaning of the described technology.

Unless the context clearly requires otherwise, throughout the description, the words "comprise," "comprising," and the like are to be construed in an inclusive sense as opposed to an exclusive or exhaustive sense; that is to say, in a sense of "including, but not limited to." Words using the singular or plural number also include the plural or singular number, respectively. Use of the word "or" in reference to a list of two or more items covers all of the following interpretations of the word: any of the items in the list, all of the items in the list, and any combination of the items in the list. Furthermore, the phrase "at least one of A, B, and C, etc." is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.).

Any patents, applications and other references, including any that may be listed in accompanying filing papers, are incorporated herein by reference. Aspects of the described technology can be modified, if necessary, to employ the systems, functions, and concepts of the various references described above to provide yet further embodiments. These and other changes can be made in light of the above Detailed Description. While the above description details certain embodiments and describes the best mode contemplated, no matter how detailed, various changes can be made. Implementation details may vary considerably, while still being encompassed by the technology disclosed herein. As noted above, particular terminology used when describing certain features or aspects of the technology should not be taken to imply that the terminology is being redefined herein to be restricted to any specific characteristics, features, or aspects of the technology with which that terminology is associated.
The invention will now be defined by reference to the following clauses:
1. An apparatus for treating a subject's submental tissue, comprising:
   a thermally conductive cup including a first sidewall, a second sidewall, and a bottom;
   a tissue-receiving cavity;
   at least one vacuum port in fluid communication with the tissue-receiving cavity to provide a vacuum for drawing the submental tissue into the tissue-receiving cavity, wherein the tissue-receiving cavity is sufficiently shallow to allow the subject's submental tissue to occupy substantially the entire tissue-receiving cavity when the vacuum is drawn via the at least one vacuum port; and
   a thermal device in thermal communication with the conductive cup, wherein the thermal device is configured to cool the conductive cup such that the first sidewall, the second sidewall, and the bottom together non-invasively cool subcutaneous lipid-rich cells in the submental tissue an amount sufficient to be biologically effective in damaging and/or reducing the subcutaneous lipid-rich cells.
2. The apparatus of clause 1 wherein each of the first sidewall, the second sidewall, and the bottom is positioned to absorb heat from the submental tissue to damage and/or reduce the lipid-rich cells, which are in a subcutaneous layer of adipose tissue, in number and/or size to an extent while non-lipid-rich cells deeper than the subcutaneous layer of adipose tissue are not reduced in number and/or size to the extent.
3. The apparatus of clause 1, further comprising
   a pressurization device in fluid communication with the tissue-receiving cavity via the vacuum port; and
   a controller with instructions for causing the apparatus to hold the submental tissue in the tissue-receiving cavity using suction provided by the pressurization device while the conductive cup conductively cools the subject's tissue.
4. The apparatus of clause 1 wherein the conductive cup includes a metal surface that faces the tissue-receiving cavity and has an area equal to or less than about 40 cm².
5. The apparatus of clause 1 wherein the tissue-receiving cavity has a length between opposing end walls of the conductive cup, a width between the first and second sidewalls, and a depth between an opening of the tissue-receiving cavity and the bottom of the conductive cup, wherein the depth is substantially uniform along most of the length of the tissue-receiving cavity.
6. The apparatus of clause 1, further comprising a restraint apparatus configured to hold a subject's head, wherein the restraint apparatus includes an adjustable pillow including a head cradle portion and being operable to controllably adjust tilt of the subject's head supported by the head cradle portion; and
   at least one restraint coupleable to the pillow such that the at least one restraint holds the conductive cup in thermal contact with the subject's submental region while the subject's head is supported by the head cradle portion.
7. An apparatus for treating a subject's tissue, comprising:
   a submental vacuum applicator including
   a tissue-receiving cavity,
   a contoured lip defining a mouth of the tissue-receiving cavity and including a first arcuate lip portion and a second arcuate lip portion, wherein the contoured lip is configured to engage a submental area of the subject such that mostly submental tissue of the subject extends through the mouth and fills substantially all of the tissue-receiving cavity while the submental vacuum applicator draws a vacuum and the first and second arcuate lip portions surround at least a portion of the subject's body, and
   a thermal device positioned to be in thermal contact with the submental tissue in the tissue-receiving cavity such that the thermal device is operable to non-invasively cool subcutaneous lipid-rich cells in the submental tissue an amount sufficient to be biologically effective in damaging and/or reducing the subcutaneous lipid-rich cells.
8. The apparatus of clause 7, further comprising a controller with instructions for causing the thermal device to cool a conductive cup of the submental vacuum applicator such that the submental vacuum applicator non-invasively cools the subcutaneous lipid-rich cells to a temperature less than about 0°C.
9. The apparatus of clause 7 wherein the tissue-receiving cavity includes a first end, a second end, and a central section extending between the first and second ends, and wherein the central section has a curved longitudinal axis and a substantially uniform maximum depth along most of the curved longitudinal axis.
10. The apparatus of clause 9 wherein the curved longitudinal axis has the same curvature as a curvature of at least one of the first and second arcuate lip portions.
11. The apparatus of clause 7 wherein the tissue-receiving cavity has a substantially uniform maximum depth along most of a longitudinal length of the tissue-receiving cavity.
12. The apparatus of clause 7, further comprising a vacuum source fluidically coupled to the tissue-receiving cavity and configured to provide sufficient vacuum to draw the submental tissue to a bottom of the tissue-receiving cavity to bring the submental tissue into thermal contact with a concave metal heat-exchanging surface of the submental vacuum applicator.
13. The apparatus of clause 7 wherein the submental vacuum applicator includes an applicator unit and a liner assembly removably attached to the applicator unit.
14. A method of non-invasively cooling a submental region of a subject, the method comprising:
   placing a submentum applicator on the subject, wherein the submentum applicator includes a vacuum cup and a tissue-receiving cavity;
   drawing submental tissue through the tissue-receiving cavity and into thermal contact with a section of the vacuum cup located at a bottom of the tissue-receiving cavity; and
   conductively extracting heat from the submental tissue to the submentum applicator so as to cool the submental tissue an amount sufficient to be biologically effective in selectively damaging and/or reducing subcutaneous submental lipid-rich cells.
15. The method of clause 14 wherein conductively extracting heat from the submental tissue causes damage to and/or reduction of a sufficient amount of the submental subcutaneous lipid-rich cells to visibly reduce a double chin of the subject.
16. The method of clause 14, further comprising
   supporting the subject's head on an adjustable pillow, wherein the adjustable pillow includes a deployable head cradle portion and is capable of controllably adjusting the forward tilt of a subject's head supported by the head cradle portion; and
   holding the submentum applicator in thermal contact with the submental region using at least one restraint coupled to the pillow.
17. A system for treating a subject, comprising:
   a restraint apparatus configured to hold a subject's head and including
   an adjustable pillow including a head cradle portion, wherein the adjustable pillow is operable to controllably adjust tilt of the subject's head supported by the head cradle portion; and
   at least one restraint coupleable to the adjustable pillow such that the at least one restraint holds a tissue-cooling apparatus in thermal contact with the subject's submental region while the subject's head is supported at a desired angle by the head cradle portion
18. The system of clause 17, further comprising the tissue-cooling apparatus, wherein the at least one restraint and the adjustable pillow are configured to cooperate to inhibit movement of the tissue-cooling apparatus relative to the subject's submental region while the tissue-cooling apparatus non-invasively cools subcutaneous lipid-rich cells at the subject's submental region an amount sufficient to be biologically effective in damaging and/or reducing the subcutaneous lipid-rich cells.
19. The system of clause 17 wherein the restraint apparatus further comprises
   a head adjuster device operable to reconfigure the head cradle portion to achieve the desired tilt of the subject's head; and
   a neck adjuster device independently operable to reconfigure a neck support portion of the adjustable pillow to achieve desired neck tilt of the subject.
20. The system of clause 17 wherein the restraint apparatus further includes
   a cradle adjuster device with a first expandable element that expands a sufficient amount to tilt the head cradle portion and increase forward tilt of the subject's head; and
   a neck adjuster device with a second expandable element that independently expands so as to cause a neck support portion of the pillow to push against a posterior region of the subject's neck.
21. The system of clause 17 wherein the adjustable pillow includes a shoulder support portion and a neck support portion positioned between the shoulder support portion and the head cradle portion, wherein the neck support portion is movable relative to the shoulder support portion and the head cradle portion such that the neck support portion pushes against the posterior region of the subject's neck.

## Claims

1. An apparatus for treating a subject's tissue, comprising:
a submental vacuum applicator including
a tissue-receiving cavity,
a contoured lip defining a mouth of the tissue-receiving cavity and including a first arcuate lip portion and a second arcuate lip portion, wherein the contoured lip is configured to engage a submental area of the subject such that mostly submental tissue of the subject extends through the mouth and fills substantially all of the tissue-receiving cavity while the submental vacuum applicator draws a vacuum and the first and second arcuate lip portions surround at least a portion of the subject's body, and
a thermal device positioned to be in thermal contact with the submental tissue in the tissue-receiving cavity such that the thermal device is operable to non-invasively cool subcutaneous lipid-rich cells in the submental tissue an amount sufficient to be biologically effective in damaging and/or reducing the subcutaneous lipid- rich cells,
wherein the tissue-receiving cavity includes a first end, a second end, and a central section extending between the first and second ends, and wherein the central section has a curved longitudinal axis and a substantially uniform maximum depth along most of the curved longitudinal axis.

2. The apparatus of claim 1, further comprising a controller with instructions for causing the thermal device to cool a conductive cup of the submental vacuum applicator such that the submental vacuum applicator non-invasively cools the subcutaneous lipid-rich cells to a temperature less than about 0°C.

3. The apparatus of claim 1 wherein the curved longitudinal axis has the same curvature as a curvature of at least one of the first and second arcuate lip portions.

4. The apparatus of claim 1 wherein the tissue-receiving cavity has a substantially uniform maximum depth along most of a longitudinal length of the tissue-receiving cavity.

5. The apparatus of claim 1, further comprising a vacuum source fluidically coupled to the tissue-receiving cavity and configured to provide sufficient vacuum to draw the submental tissue to a bottom of the tissue-receiving cavity to bring the submental tissue into thermal contact with a concave metal heat-exchanging surface of the submental vacuum applicator.

6. The apparatus of claim 1 wherein the submental vacuum applicator includes an applicator unit and a liner assembly removably attached to the applicator unit.
